# EUROPÄISCHE PATENTSCHRIFT

(11) **EP 2 240 457 B1**
(45) Veröffentlichungstag und Bekanntmachung des Hinweises auf die Patenterteilung: **11.05.2011**
(21) Anmeldenummer: 08877913.7
(22) Anmeldetag: 02.12.2008
(51) Int. Cl.: C07D 277/32

(54) **NEUE 2-ARYL-THIAZOL-4-CARBONSÄUREAMID-DERIVATE, DEREN HERSTELLUNG UND VERWENDUNG ALS ARZNEIMITTEL**
NOVEL 2-ARYL-THIAZOLE-4-CARBOXAMIDE DERIVATIVES, PRODUCTION THEREOF, AND USE THEREOF AS A MEDICAMENT
NOUVEAUX DERIVES DE 2-ARYL-THIAZOLE-4-CARBOXAMIDE, LEUR PRODUCTION ET L'UTILISATION MEDICALE

(30) Priorität: 10.12.2007 EP 07076069
(43) Veröffentlichungstag der Anmeldung: 20.10.2010
(73) Patentinhaber: Bayer Schering Pharma Aktiengesellschaft, 13353 Berlin (DE)
(72) Erfinder: BOTHE, Ulrich, 13187 Berlin (DE); VON BONIN, Arne, 16548 Glienicke/Nordbahn (DE); NGUYEN, Duy, 10179 Berlin (DE); BÖMER, Ulf, 16548 Glienecke (DE); GUENTHER, Judith, 13187 Berlin (DE)
(86) Internationale Anmeldenummer: PCT/EP2008/010188
(87) Internationale Veröffentlichungsnummer: WO 2009/074247

(56) Entgegenhaltungen:
- WO-A-2007/035478
- WO-A-2007/123269
- DATABASE CHEMCATS [Online] Chemical Abstracts; 23. Oktober 2007 (2007-10-23), XP002478677 gefunden im STN Database accession no. 2040796643

## Beschreibung

Die vorliegende Erfindung betrifft 2-Aryl-thiazol-4-carbonsäureamid-Derivate und deren Verwendung zur Herstellung eines Medikaments zur Behandlung verschiedener Erkrankungen.

### Biologischer Hintergrund

Für zahlreiche chronisch entzündliche Erkrankungen, wie z.B. Rheumatoider Arthritis, Morbus Crohn, Asthma und Multipler Sklerose, ist ein überreagierendes Immunsystem mit verantwortlich. Durch ein vermehrtes Freisetzen von proinflammatorischen Zytokinen kommt es zu Schädigungen von körpereigenen Gewebestrukturen. Hierbei ist das Zusammenspiel von angeborenem und adaptiven Immunsystem von zentraler Bedeutung (Akira et al., 2001). Eine Modulation des Immunsystems mit Substanzen, die mit der Aktivierung von Zellen des angeborenen und/oder des adaptiven Immunsystem interferieren, wirkt anti-inflammatorisch und kann damit den pathologischen Phänotyp in den oben exemplarisch genannten Erkrankungen abmildern.
Die angeborene Immunität ("innate immunity") beruht darauf, daß Mikroorganismen wie Bakterien und Viren bestimmte inherente Merkmale aufweisen, durch die sie vom Immunsystem erkannt werden und dieses anschließend aktivieren. Erkannt werden bestimmte Pathogen assozierte Muster ("pathogen associated molecular pattern, PAMPs"). PAMPs werden von den "Pattern recognition receptors" (PRR) erkannt, zu denen auch Toll Like Rezeptoren (TLR) gehören (Janeway und Medzhitov, 2002). TLRs sind Homologe zum Drosophila-Rezeptorprotein "toll". Der Mensch hat zehn verschiedene TLRs. TLR eins und sechs sind Corezeptoren für TLR2. TLR2 erkennt u.a. Lipoproteine und Lipopeptide. TLR3 erkennt doppelsträngige RNA. TLR4 erkennt u.a. LPS von gram-negativen und Lipoteichonsäure von gram-positiven Bakterien. TLR5 erkennt Flagellin. TLR9 erkennt CpG-Motive in bakterieller DNA (O'Neill, 2006). Corezeptoren können die Erkennungsfähigkeiten von TLRs weiter verändern (Jiang et al., 2005).

### IL-1/-18, TLR Signaltransduktion

TLRs sind in der Signalübertragung mit IL-1 / IL-18 Zytokinrezeptoren verwandt. IL-1 ("endogenes Pyrogen") stimuliert stark Entzündung und induziert Fieber.
Mitglieder der IL-1R/TLR Superfamilie haben eine TIR Domäne (Toll/IL1 Receptor). Die TIR Domäne ist etwa 200 Aminosäuren lang und enthält drei konservierte Sequenzmotive. TIR-Domänen tragende Proteine binden über eine Protein-Protein Interaktion (O'Neill et al., 2005). Die Subklasse eins (IL-1R Familie) enthält drei Igartige-Domänen, der Rezeptor ist ein Heterodimer. Dazu gehören die IL-1 Rezeptoren eins und zwei, der Co-Rezeptor IL-1RAcP und die entsprechenden Proteine des IL-18 Systems. Die Subklasse zwei (TLR-Familie) enthält Leucin-rich Motife. Toll-like Rezeptoren bilden Homo- oder Heterodimere.

Nach Aktivierung der TLR bzw. IL-1, -18 Rezeptoren durch die entsprechenden Liganden wird eine mehrstufige Signalkaskade in Gang gesetzt. Der TLR bzw. IL-1/- 18 Rezeptorkomplex wechselwirkt über TIR/TIR Kontakte mit dem Adaptorprotein MyD88. Die "IL-1 associated receptor kinase" (IRAK-1) hat normalerweise Tollip (Toll interacting protein) gebunden, das wahrscheinlich als ein abschwächendes Molekül ("silencer") wirkt. IRAK/Tollip bindet an den aktiven TLR/IL-1R Komplex. MyD88 verdrängt Tollip wodurch IRAK1 und IRAK-4 aktiviert wird, höchstwahrscheinlich als Dimer durch Transphosphorylierung. Aktives IRAK verlässt den Rezeptor und bindet im Cytoplasma an das Adaptermolekül TRAF (Barton und Medzhitov, 2003). Über TRAF werden weitere Proteine ubiquitiniliert. Über einen unbekannten Mechanismus führt Ub-TRAF zur Autophosphorylierung der S/T-Kinase TAK1 (eine MAP-Kinase Kinasekinase). TAK1 phosphoryliert IκB (NF-κB Aktivierung) und MKK6. Letztere ist für die Aktivierung der MAP-Kinasen p38 und JNK verantwortlich. NF-κB wurde als Nuclear Factor für die Expression der leichten Antikörperkette Kappa in B-Zellen identifiziert, ist aber an der Regulation vieler anderer Gene ebenfalls beteiligt. NF-κB wird im inaktiven Zustand im Cytoplasma zurückgehalten, wo es an den Inhibitor IκB gebunden ist (Deng et al., 2000). Phosphorylierung von IκB bewirkt, daß der Inhibitor IkB proteolytisch abgebaut wird und der Transkriptionsfaktor in den Kern wandern kann. NF-κB ist ein Heterodimer aus den Untereinheiten p65 (Rel) und p50 (Bäuerle und Henkel, 1994). Es gibt mehrere Mitglieder dieser Familie, die auf unterschiedliche Weise zusammenwirken können. NF-κB alleine kann Transkription nicht auslösen. Für Genaktivierung sind transkriptionelle Coaktivatoren erforderlich, wie etwa p300 oder CBT (Akira und Takeda, 2004).
Nach erfolgter Aktivierung von TIR-Domäne enthaltenden Rezeptoren kommt es unter anderem zur Freisetzung von inflammatorischen Zytokinen, wie z.B. IL-1, IL-6, IL-23 und TNF-alpha (Adachi et al., 1998).

Den strukturell naheliegenden Stand der Technik bilden die Strukturen folgender Patentanmeldungen:
In W02007/016292 werden N-Aryl-2-arylthiazol-4-carbonsäureamid-Derivate als Biofilmrnodulatoren (Modulatoren von Bakterienfilmen) beschrieben, die aufgrund des bicyclischen C-D-Ringsystemes unterschiedlich von den hier beanspruchten Verbindungen sind.
In W02007/035478 werden Verbindungen offenbart, die anstelle einer Carboxamidgruppe eine Carboxylgruppe in ortho-Positon aufweisen.
In US 6,274,738 werden N-Aryl-2-pyridylthiazol-4-carbonsäureamid-Derivate als Verbindungen beschrieben, die die DNA Primase modulieren. Allerdings weisen diese Verbindungen an der N-Arylgruppe keine Aminocarbonylgruppe auf, die in ortho-Postion bezüglich der Pyridylthiazol-4-carbonsäureamideinheit steht.
Darüber hinaus werden in US 4,879295 N-Tetrazolylthiazolcarboxamide offenbart. Thiazolamidderivate werden in WO2006/122011 als Inhibitoren der Virusreplikation genannt. In WO2005/048953 werden Thiazolamidderivate verknüpft mit einer Isoxazoleinheit als Kinaseinhibitoren berschrieben. Die Strukturen unterscheiden sich allerdings von den Strukturen der vorliegenden Erfindung.
Auch die in der W02007/052882 offenbarten Verbindungen weisen keine Aminocarbonylgruppe in ortho-Position zur Aminocarbonylthiazolgruppe auf. Besonderes Merkmal der in W02004072025 beschriebenen Verbindungen ist im Gegensatz zu den hier offenbarten Strukturen ein Pyrrolidinring, der zusätzlich noch mit einem weiteren Substituenten (wie z. B. einer Dimethylaminogruppe) über ein Stickstoffatom verknüpft vorliegt.

In W0200512256, W0200677424, WO2006077425 und WO200677428 werden Pyrazolderivate als Kinaseinhibitoren offenbart. Unter anderem sind auch Strukturen beschrieben, bei denen ein Thiazolamid mit der Pyrazoleinheit verknüpft ist, wobei allerdings keines der Pyrazol-Stickstoffatome methyliert vorliegt und die Pyrazoleinheit auch nicht mit einer Aminocarbonylgruppe (C(O)NH₂) substituiert ist.

Pyrazolamide, die aber gleichzeitig mit einer Harnstoffeinheit verknüpft vorliegen, werden in WO2005/37797 beschrieben.

In WO2005/115986 werden Pyridinamide beansprucht, wobei aber keine Verknüpfungen mit schwefelhaltigen Heterocyclen wie Thiazol vorgesehen sind.

Strukturell unterschiedlich zu den hier beschriebenen Verbindungen sind auch die in WO2005/049604 beschriebenen Pyridinamide, aufgrund der Verknüpfung mit einem sauerstoffsubstituierten Phenylring.

In EP1666455 werden Amide mit zusätzlicher Carboxamidstruktur beschrieben, wobei der Substituent R1 keine Aminocarbonylgruppe sein kann.

Ausgehend von diesem Stand der Technik besteht die Aufgabe der vorliegenden Erfindung, weitere Strukturen für die Therapie bereitzustellen, insbesondere für die Immunmodulation.

Die Aufgabe wird gelöst durch Verbindungen der allgemeinen Formel (I), mit den Bausteinen A, B, C und D , 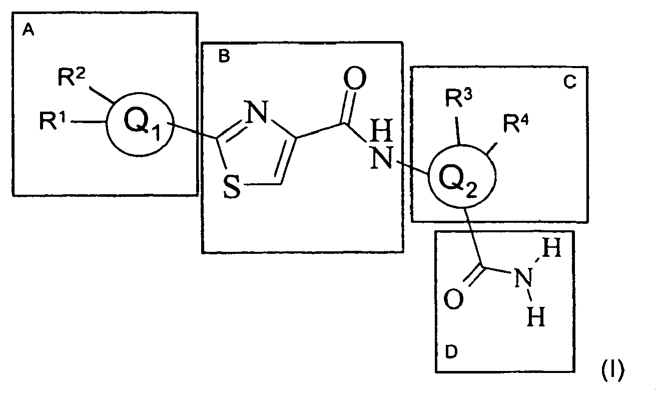 in der
die Bausteine B und D in ortho-Stellung zueinander stehen und
- Q₁: für einen Phenylring oder hydrierten bicyclischen Heteroarylring steht, und
- R¹ und R²: unabhängig voneinander stehen für
(i) Wasserstoff, Hydroxy, Nitro, Halogen, Cyano, -CF₃, -NR⁵R⁶ oder
(ii) -O-SO₂-NR⁵R⁶, -SO₂-R⁷, -SO₂-NR⁵R⁶
(iii) -C(O)-OR¹⁰, -C(O)-R⁷, -C(O)-C₁-C₃-Fluoralkyl, -C(O)-NR⁵R⁶, -NH-C(O)-R⁷ oder
(iv) einen C₂-C₆ -Alkenyl-, C₂-C₆-Alkinyl-, C₁-C₆-Alkoxy-, C₁-C₆-Fluoralkyl- oder einen C₁-C₆-Fluoralkoxyrest, oder
(v) einen C₁-C₆-Alkylrest, wenn Q₁ für einen hydrierten bicyclischen Heteroarylring steht,
wobei (iv) und/oder (v) gegebenenfalls mit -C₁-C₃-Alkoxy, Hydroxy, -C(O)-OR¹⁰ oder -NR⁸R⁹ ein- oder mehrfach, gleich oder verschieden substituiert sein können, und
- Q₂: für einen Aryl-, Heteroaryl- oder einen hydrierten bizyklischen Heteroarylring steht, und
- R³ und R⁴: unabhängig voneinander stehen für
(i) Wasserstoff, Halogen oder-NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷, und
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder - C(O)-R⁷, oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
- R⁷: für einen C₁-C₆ Alkylrest steht, und
- R⁸, R⁹, R¹⁰: unabhängig voneinander stehen für Wasserstoff oder einen C₁-C₆ Alkylrest, und
- R¹¹ und R¹²: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder - C(O)-R⁷,
sowie deren Salze, Enantiomere und Diastereomere.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, iso-Propyl-, iso-Butyl-, sec-Butyl, *tert-*Butyl-, iso-Pentyl-, 2-Methylbutyl-, 1-Methylbutyl-, 1-Ethylpropyl-, 1,2-Dimethylpropyl, neo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

### Cₙ-Fluoralkyl:

Monovalenter, geradkettiger oder verzweigter, gesättigter, vollständig oder teilweise fluorierter Kohlenwasserstoffrest mit n Kohlenstoffatomen.

Ein C₁-C₆ Fluoralkylrest umfasst unter anderem beispielsweise:
Trifluormethyl, Difluormethyl, Monofluormethyl, Pentafluorethyl, Heptafluorpropyl, 2,2,2-Trifluorethyl, 3,3,3-Trifluorpropyl, 5,5,6,6,6-Pentafluorhexyl, Pentafluorallyl, 1,1,1,3,3,3-Hexafluor-2-propyl.

Bevorzugt ist ein Trifluormethyl, 2,2,2-Trifluorethyl und Pentafluorethylrest.

### Cₙ-Alkenyl:

monovalenter, geradkettiger oder verzweigter Kohlenwasserstoffrest mit n Kohlenstoffatomen und mindestens einer Doppelbindung.

Ein C₂-C₆ Alkenylrest umfasst unter anderem beispielsweise:
Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-,
(Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-,
2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alkinyl:

Monovalenter, geradkettiger oder verzweigter Kohlenwasserstoffrest mit n Kohlenstoffatomen und mindestens einer Dreifachbindung.

Ein C₂-C₆ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.
Bevorzugt ist ein Ethinyl-, Prop-1-inyl- oder Prop-2-inylrest.

### Cₙ-Cycloalkyl:

Monovalenter, cyclischer Kohlenwasserstoffring mit n Kohlenstoffatomen.

C₃-C₇-Cyclolalkylring umfasst:
Cyclopropyl, Cyclobutyl, Cyclopentyl, Cyclohexyl- und Cycloheptyl.

Bevorzugt ist ein Cyclopropyl-, Cyclobutyl-, Cylopentyl- oder ein Cyclohexylring.

### Cₙ-Alkoxy:

Geradkettiger oder verzweigter Cₙ-Alkyletherrest der Formel -OR mit R=Alkyl.

Cₙ-Fluoralkoxy:

Geradkettiger oder verzweigter Cₙ-Fluoralkyletherrest der Formel -OR mit R=Cₙ-Fluoralkyl.

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl. C₁₀-Aryl ist gleich Naphthyl.
Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Ein monocyclischer Heteroarylring gemäß der vorliegenden Erfindung hat 5 oder 6 Ringatome.

Heteroarylringe mit 5 Ringatomen umfassen beispielsweise die Ringe:
Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylringe mit 6 Ringatomen umfassen beispielsweise die Ringe: Pyridinyl, Pyridazinyl, Pyrimidinyl, Pyrazinyl und Triazinyl.

Ein bicyclischer Heteroarylring gemäß der vorliegenden Erfindung hat 9 bis 10 Ringatome.

Heteroarylringe mit 9 Ringatomen umfassen beispielsweise die Ringe:
Indolyl, Isoindolyl, Indazolyl, Benzothiazolyl, Benzofuranyl, Benzothiophenyl, Benzimidazolyl, Benzoxazolyl, Azocinyl, Indolizinyl, Purinyl.

Heteroarylringe mit 10 Ringatomen umfassen beispielsweise die Ringe:
Isochinolinyl, Chinolinyl, Cinnolinyl, Chinazolinyl, Chinoxalinyl, Phthalazinyl, 1,7- oder 1,8-Naphthyridinyl, Pteridinyl

Monocyclische Heteroarylringe mit 5 oder 6 Ringatomen sind bevorzugt.

Hydrierte bicyclische Aryl- oder Heteroarylringe sind Bizyklen, bei denen ein Ring teilweise oder vollständig hydriert vorliegt. Die Bindungsvalenz kann an einem beliebigen Atom des aromatischen Teiles des hydrierten bicyclischen Aryl- oder Heteroarylringes sein.

Hydrierte bicyclische Aryl- oder Heteroarylringe umfassen beispielsweise die Ringe: Indanyl, 1,2,3,4-Tetrahydro-naphthalenyl, 1,2,3,4-Tetrahydro-isochinolinyl, 1,2,3,4-Tetrahydro-chinolinyl, 2,3-Dihydro-1H-indolyl, 2,3-Dihydro-1H-isoindolyl, 4,5,6,7-Tetrahydro-thieno[2,3-c]pyridinyl, 4,5,6,7-Tetrahydro-thieno[3,2-c]pyridinyl, 2,3-Dihydro-benzofuranyl, Benzo[1,3]dioxolyl, 2,3-Dihydro-benzo[1,4]dioxinyl, Chromanyl, 1,2,3,4-Tetrahydro-chinoxalinyl, 3,4-Dihydro-2H-benzo[1,4]oxazinyl.

Hydrierte bicyclische Aryl- oder Heteroarylringe bei denen ein Ring teilweise oder vollständig hydriert vorliegt, können gegebenenfalls eine oder zwei Carbonylgruppen im Ringsystem enthalten.
Beispiele hierfür sind Indolonyl, Isoindolonyl, Benzoxazinonyl, Phthalazinonyl, Chinolonyl, Isochinolonyl, Phthalidyl, Thiophthalidyl.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein.

Heterocycylring mit 3 Ringatomen umfasst beispielsweise:
Aziridinyl.

Heterocycylring mit 4 Ringatomen umfasst beispielsweise:
Azetidinyl, Oxetanyl.

Heterocycylringe mit 5 Ringatomen umfassen beispielsweise die Ringe:
Pyrrolidinyl, Imidazolidinyl, Pyrazolidinyl und Tetrahydrofuranyl.

Heterocyclylringe mit 6 Ringatomen umfassen beispielsweise die Ringe:
Piperidinyl, Piperazinyl, Morpholinyl, Tetrahydropyranyl und Thiomorpholinyl

Heterocycylringe mit 7 Ringatomen umfassen beispielsweise:
Azepanyl, Oxepanyl, [1,3]-Diazepanyl, [1,4]-Diazepanyl.

Heterocycylringe mit 8 Ringatomen umfassen beispielsweise:
Oxocanyl, Azocanyl.

Heterocyclylringe können gegebenenfalls teilweise ungesättigt sein und/oder auch eine Carbonylgruppe im Ring enthalten.
Beispiele hierfür sind Dihydro-furan-2-onyl, Pyrrolidin-2-onyl, Piperazin-2-onyl, Morpholin-2-onyl, 3(2H)-Pyridazinonyl, 5,6-Dihydro-2-pyran-2-onyl, 5,6-Dihydropyridin-2(1H)-onyl, 2-Piperidonyl, 1,2,3,6-Tetrahydropyridinyl.

### Halogen

Die Bezeichnung Halogen umfasst Fluor, Chlor, Brom und Iod.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Ebenfalls als von der vorliegenden Erfindung als erfasst anzusehen sind die Salze der Verbindungen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Aufgrund ihrer anti-entzündlichen und zusätzlichen immunsuppressiven Wirkung können die erfindungsgemäßen Verbindungen der allgemeinen Formel (I) als Medikamente zur Behandlung oder Prophylaxe folgender Krankheitszustände bei Säugetieren und Menschen, für die lokale und systemische Applikation Verwendung finden:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS), akutes Atemnotssyndrom
   - Bronchiektasen
   - Alle Formen der restriktiven Lungenerkrankungen, vor allem allergische Alveolitis,
   - Lungenödem, insbesondere allergisches
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Weichteilrheumatismus
(iii) Allergien oder pseudoallergische Erkrankungen, die mit entzündlichen, und /oder proliferativen Prozessen einhergehen:
   - alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Riesenzellarteriitis
(v) Dermatologische Erkrankungen, die mit entzündlichen, allergischen und / oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimmuner Pemphigus vulgaris, bullöses Pemphigoid
   - Erkrankungen des lichenoiden Formenkreises,
   - Pruritus (z. B. allergischer Genese)
   - Rosacea-Formenkreis
   - Erythema exsudativum multiforme
   - Manifestation von Gefäßerkrankungen
   - Haarausfall wie Alopecia areata - Kutane Lymphome
(vi) Nierenerkrankungen, die mit entzündlichen, allergischen und / oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akute Hepatitis unterschiedlicher Genese
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastroenteritiden anderer Genese, z.B. einheimische Sprue
(ix) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioiditis
   - Ophthalmia sympathica
(x) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktekzem
(xi) Neurologische Erkrankungen, die mit entzündlichen, allergischen und / oder proliferativen Prozessen einhergehen:
   - Hirnödem, vor allem allergisches Hirnödem
   - Multiple Sklerose
   - akute Encephalomyelitis
   - Meningitis, vor allem allergische
   - Guillain-Barre Syndrom
   - Morbus Alzheimer
(xii) Bluterkrankungen, die mit entzündlichen, allergischen und / oder proliferativen Prozessen einhergehen wie z. B.: M. Hodgkin oder Non-Hodgkin Lymphome, Thrombozytaemien, Erythrozytosen
   - Erworbene hämolytische Anämie
   - Idiopathische Thrombozytopenie
   - Idiopathische Granulozytopenie
(xiii) Tumorerkrankungen, die mit entzündlichen, allergischen und / oder proliferativen Prozessen einhergehen
   - Akute lymphatische Leukämie
   - Maligne Lymphome
   - Lymphogranulomatosen
   - Lymphosarkome
(xiv) Endokrine Erkrankungen, die mit entzündlichen, allergischen und / oder proliferativen Prozessen einhergehen wie z. B.:
   - Endokrine Orbitopathie
   - Thyreoiditis de Quervain
   - Hashimoto Thyreoiditis
   - Morbus Basedow
   - Granulomatous thyroiditis
   - Struma lymphomatosa
   - Autoimmune adrenalitis
   - Diabetes mellitus, insbesondere Typ 1 Diabetes
   - Endometriose
(xv) Organ- und Gewebstransplantationen, Graft-versus-host-disease
(xvi) Schwere Schockzustände, z.B anaphylaktischer Schock, systemic inflammatory response syndrome (SIRS)

Ein Gegenstand der Erfindung ist die Verwendung der erfindungsgemäßen Verbindungen der allgemeinen Formel (I) zur Herstellung eines Arzneimittels.

Ein weiterer Gegenstand der Erfindung sind die erfindungsgemäßen Verbindungen zur Behandlung von Erkrankungen, die mit entzündlichen, allergischen und / oder proliferativen Prozessen einhergehen.

In der allgemeinen Formel (I) kann Q₁ für einen Phenylring oder hydrierten bicyclischen Heteroarylring stehen.

Bevorzugt steht Q₁ für einen für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl-, Indanonyl- oder einen 2,3-Dihydroindolylring.

Besonders bevorzugt steht Q₁ für einen Phenyl-, 3,4-Methylendioxyphenyl-, 2,3-Dihydrobenzofuranyl- oder einen 3,4-Dihydro-2H-benzo[1,4]oxazin-7-ylring.

In der allgemeinen Formel (I) können R¹ und R² unabhängig voneinander stehen für:
(i) Wasserstoff, Hydroxy, Nitro, Halogen, Cyano, -CF₃, -NR⁵R⁶ oder
(ii) -O-SO₂-NR⁵R⁶, -SO₂-R⁷, -SO₂-NR⁵R⁶
(iii) -C(O)-OR¹⁰, -C(O)-R⁷, -C(O)-C₁-C₃-Fluoralkyl, -C(O)-NR⁵R⁶, -NH-C(O)-R⁷ oder
(iv) einen C₂-C₆ -Alkenyl-, C₂-C₆-Alkinyl-, C₁-C₆-Alkoxy-, C₁-C₆-Fluoralkyl- oder einen C₁-C₆-Fluoralkoxyrest, oder
(v) einen C₁-C₆-Alkylrest, wenn Q₁ für einen hydrierten bicyclischen Heteroarylring steht,
   wobei (iv) und/oder (v) gegebenenfalls mit -C₁-C₃-Alkoxy, Hydroxy, -C(O)-OR¹⁰ oder -NR⁸R⁹ ein- oder mehrfach, gleich oder verschieden substituiert sein können.

Bevorzugt stehen R¹ und R² unabhängig voneinander für:
(i) Wasserstoff, Hydroxy, Halogen, Cyano, -CF₃, -NR⁵R⁶ oder
(ii) -SO₂-R⁷,
(iii) einen C₁-C₆-Alkoxy-, C₁-C₆-Fluoralkyl-, C₁-C₆-Fluoralkoxyrest oder
(iv) einen C₁-C₆-Alkylrest, wenn Q₁ für einen hydrierten bicyclischen Heteroarylring steht.

Besonders bevorzugt stehen R¹ und R² unabhängig voneinander für:
(i) Wasserstoff, Halogen, Cyano oder
(ii) -SO₂-R⁷.
(iii) einen C₁-C₆-Alkoxyrest oder
(iv) einen C₁-C₆-Alkylrest, wenn Q₁ für einen hydrierten bicyclischen Heteroarylring steht.

In der allgemeinen Formel (I) kann Q₂ stehen für einen Aryl-, Heteroaryl- oder einen hydrierten bizyklischen Heteroarylring.

Bevorzugt steht Q₂ für einen:
Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring.

Besonders bevorzugt steht Q₂ für einen Phenyl-, Pyrazolyl- oder einen 4,5,6,7-Tetrahydro-thieno[2,3-c]pyridinylring.

In der allgemeinen Formel (I) können R³ und R⁴ unabhängig voneinander stehen für:
(i) Wasserstoff, Halogenoder-NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷.

Bevorzugt stehen R³ und R⁴ unabhängig voneinander für:
(i) Wasserstoff, Halogen, -NR¹¹R¹² oder
(ii) einen C₁-C₆-Alkyl- oder C₁-C₆-Alkoxyrest,
   jeweils gegebenenfalls mit Morpholin oder -NR⁸R⁹ substituiert.

Besonders bevorzugt stehen R³ und R⁴ unabhängig voneinander für Wasserstoff, Halogen oder C₁-C₆-Alkyl.

In der allgemeinen Formel (I) können R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist.

Bevorzugt stehen R⁵ und R⁶ unabhängig voneinander für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann.

Besonders bevorzugt stehen R⁵ und R⁶ unabhängig voneinander für Wasserstoff oder einen C₁-C₄-Alkylrest.

In der allgemeinen Formel (I) kann R⁷ stehen für einen C₁-C₆ Alkylrest.

Bevorzugt steht R⁷ für einen C₁-C₄-Alkylrest.

Besonders bevorzugt steht R⁷ für einen C₁-C₃-Alkylrest.

In der allgemeinen Formel (I) können R⁸, R⁹, R¹⁰ unabhängig voneinander stehen für Wasserstoff oder einen C₁-C₆-Alkylrest.

Bevorzugt stehen R⁸ und R⁹ unabhängig voneinander für Wasserstoff oder einen C₁-C₄-Alkylrest und R¹⁰ für Wasserstoff.

Besonders bevorzugt stehen R⁸ und R⁹ unabhängig voneinander für Wasserstoff oder einen C₁-C₃-Alkylrest und R¹⁰ für Wasserstoff

In der allgemeinen Formel (I) können R¹¹und R¹² unabhängig voneinander stehen für: Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,

Bevorzugt stehen R¹¹ und R¹² unabhängig voneinander für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann.

Besonders bevorzugt stehen R¹¹ und R¹² unabhängig voneinander für Wasserstoff oder einen C₁-C₆ Alkylrest

Eine bevorzugte Untergruppe bilden Verbindungen der Formel (I) mit den Bausteinen A, B, C und D,
in der die Bausteine B und D in ortho-Stellung zueinander stehen und
- Q₁: für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl-, Indanonyl- oder einen 2,3-Dihydroindolylring steht,
- R¹ und R²: unabhängig stehen für
(i) Wasserstoff, Hydroxy, Halogen, Cyano, -CF₃, -NR⁵R⁶ oder
(ii) -SO₂-R⁷,
(iii) einen C₁-C₆-Alkoxy-, C₁-C₆-Fluoralkyl-, C₁-C₆-Fluoralkoxyrest oder
(iv) einen C₁-C₆-Alkylrest, wenn Q₁ für einen hydrierten bicyclischen Heteroarylring steht,
- Q₂: für einen Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht,
- R³ und R⁴: unabhängig voneinander stehen für
(i) Wasserstoff, Halogen, -NR¹¹R¹² oder
(ii) einen C₁-C₆-Alkyl- oder C₁-C₆-Alkoxyrest,
jeweils gegebenenfalls mit Morpholin oder -NR⁸R⁹ substituiert, wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann, und
R⁷ für einen C₁-C₄ Alkylrest steht, und
R⁸ und R⁹ unabhängig voneinander stehen für Wasserstoff oder einen C₁-C₄ Alkylrest,
R¹¹ und R¹² unabhängig voneinander stehen für Wasserstoff oder einen C₁-C₆ Alkylrest, der gegebenenfalls ein- oder mehrfach, gleich oder verschieden mit Morpholin oder durch -N(CH₃)₂. -NH-CH₃ oder -NH-C₂H₅ substituiert sein kann,
sowie deren Salze, Enantiomere und Diastereomere.

Besonders bevorzugt sind Verbindungen der allgemeinen Formel (I) mit den Bausteinen A, B, C und D,
in der
die Bausteine B und D in ortho-Stellung zueinander stehen und
- Q₁: für einen für Phenyl-, 3,4-Methylendioxyphenyl-, 2,3-Dihydrobenzofuranyl- oder einen 3,4-Dihydro-2H-benzo[1,4]oxazin-7-ylring steht, und
- R¹ und R²: unabhängig voneinander stehen für
(i) Wasserstoff, Halogen, Cyano oder
(ii) -SO₂-R⁷.
(iii) einen C₁-C₆-Alkoxyrest oder
(iv) einen C₁-C₆-Alkylrest, wenn Q₁ für einen hydrierten bicyclischen Heteroarylring steht, und
- Q₂: für einen für einen Phenyl-, Pyrazolyl- oder einen 4,5,6,7-Tetrahydro-thieno[2,3-c]pyridinylring steht, und
- R³ und R⁴: unabhängig voneinander stehen für Wasserstoff, Halogen oder C₁-C₆-Alkyl,
wobei
R⁷ für einen C₁-C₃-Alkylrest steht,
sowie deren Salze, Enantiomere und Diastereomere.

### Herstellung der erfindungsgemäßen Verbindungen

### Verfahrensvariante 1:

### Herstellung der erfindungsgemäßen Verbindungen durch Amidkupplung Endproduktes

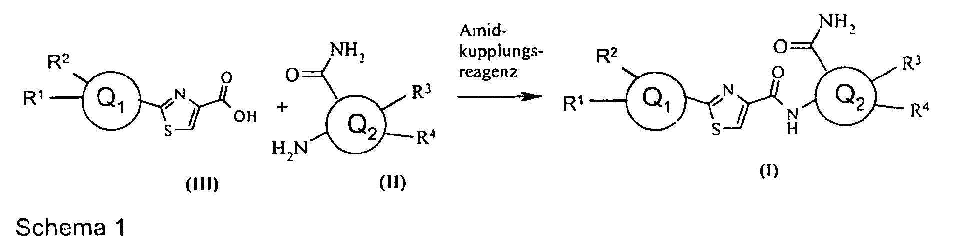

Die Darstellung der erfindungsgemäßen Verbindungen der allgemeinen Formel (I) kann durch Umsetzung der Intermediate gemäß Formel (III) mit Verbindungen der Fomel II erfolgen durch ein Amidkupplungsreagenz (siehe Publikation Chemfiles, Peptide Synthesis, 2007, 7, Nr. 2 der Firma Sigma-Aldrich). Hierbei können zum Beispiel Carbodiimide (zum Beispiel N-Cyclohexyl-N'-(2-morpholinoethyl)carbodiimid metho-p-toluolsulfonat CAS2491-17-0) oder Uroniumsalze (zum Beispiel O-(7-Azabenzotriazol-1-yl)-N,N,N'N'-tetramethyluroniumhexafluorophosphat (HATU)) verwendet werden in Kombination mit einer Base wie zum Beispiel Pyridin.
Hierbei haben Q₁, Q₂, R¹, R², R³ und R⁴ die in der allgemeinen Formel (I) gemäß der Ansprüche 1 bis 7 angegebenen Bedeutungen.

### Verfahrensvariante 2:

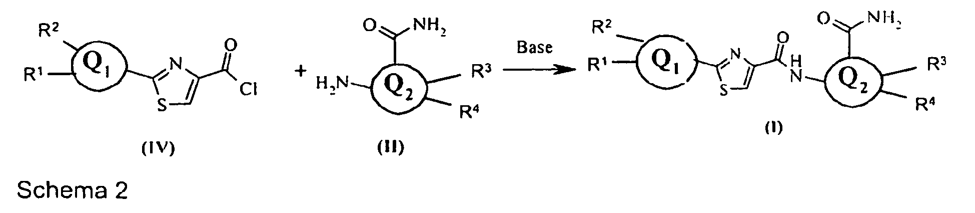

Die Darstellung der erfindungsgemäßen Verbindungen der allgemeinen Formel (I) erfolgt bei dieser Variante durch die Umsetzung der Intermediate gemäß Formel (IV) mit Verbindungen der Fomel (II) in Gegenwart einer Base wie zum Beispiel Triethylamin. Hierbei haben Q₁, Q₂, R¹, R², R³ und R⁴ die in der allgemeinen Formel (I) gemäß der Ansprüche 1 bis 7 angegebenen Bedeutungen.

### Verfahrensvariante 3:

### a) Herstellung von Intermediaten der Formel (V)

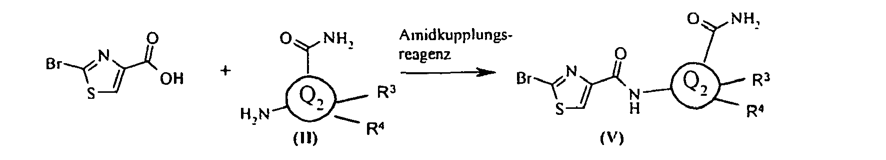

2-Bromthiazol-4-carbonsäure wird mit Intermediaten der Formel (II) durch ein Amidkupplungsreagenzien wie bei Verfahrensvariante 1 beschrieben zu Intermediaten der Formel (V) umgesetzt.

### b) Herstellung des Endproduktes

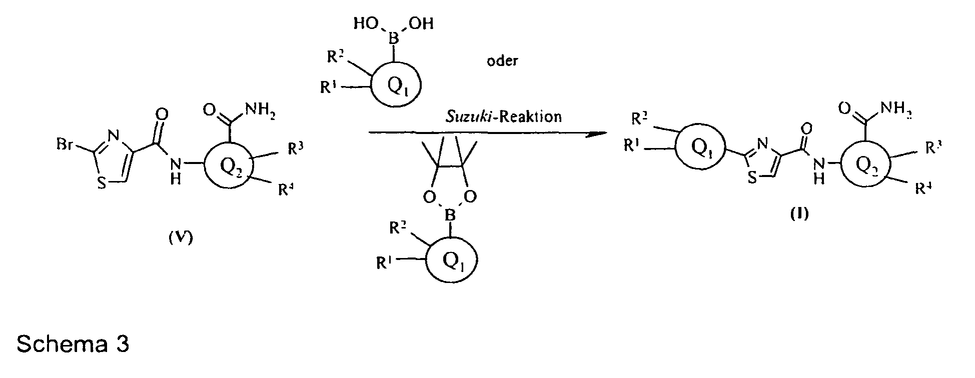

Intermediate der Formel (V) werden mit Boronsäuren oder Boronsäurepinakolestern im Rahmen einer *Suzuki-Miyaura-Reaktion* zu den erfindungsgemäßen Verbindungen der Formel (I) umgesetzt. Als Katalysatoren bzw. Liganden kommen dabei zum Beispiel die in N. Miyaura, A. Suzuki, Chem. Rev.; 1995; 95(7); 2457-2483 oder in C. J. O'Brien et. al. Chem. Eur. J. 2006, 12, 4743 - 4748 sowie die in der Publikation Chemfiles, Catalysis, 2007, 7, Nr. 5 von der Firma Sigma-Aldrich beschrieben Katalysatoren bzw. Katalysatorensysteme in Betracht.
Hierbei haben Q₁, Q₂, R¹, R², R³ und R⁴ die in der allgemeinen Formel (I) gemäß der Ansprüche 1 bis 7 angegebenen Bedeutungen.

### Reinigung der erfindungsgemäßen Verbindungen

In einigen Fällen können die erfindungsgemäßen Verbindungen durch präparative HPLC zum Beispiel durch ein Autopurifier-Gerät der Firma Waters (Detektion der Verbindungen durch UV-Detektion sowie Elektrospray-lonisation) in Kombination mit kommerziell erhältlichen, vorgepackten HPLC Säulen (zum Beispiel Säule XBridge (Firma Waters), C18, 5µm, 30 x 100mm) gereinigt werden. Als Lösemittelsystem kann Acetonitril / Wasser +0,1 % Trifluoressigsäure verwendet werden (Fluss 50 ml /min). Zum Entfernen des HPLC-Lösemittelgemisches kann eine Gefriertrocknung oder eine Zentrifugation erfolgen. Die so erhaltenen Verbindungen können als Trifluoressigsäure (TFA)-Salze vorliegen und können zu den jeweiligen freien Basen durch dem Fachmann bekannte Standard-Laborprozeduren überführt werden.
In einigen Fällen können die erfindungsgemäßen Verbindungen durch Chromatographie an Kieselgel gereinigt werden. Hierbei können zum Beispiel vorgepackte Silica Gel Cartridges (zum Beispiel von der Firma Separtis, *Isolute® Flash silica gel*) in Kombination mit dem Flashmaster II Chromatograhiegerät (Argonaut/Biotage) und Chromatographielösemittel bzw. -Gemische wie zum Beispiel Hexan, Essigester sowie Dichlormethan und Methanol in Betracht.

### Strukturanalytik der erfindungsgemäßen Verbindungen:

In einigen Fällen werden die erfindungsgemäßen Verbindungen durch LC-MS analysiert: Retentionszeiten R₁ aus der LC-MS-Analytik: Detektion: UV = 200 - 400 nm *(Acquity* HPLC-System der Firma *Waters)*/ MS 100-800 Daltons; 20 V (Micromass / *Waters* ZQ 4000) im ESI⁺-Modus (zur Erzeugung positiv geladener Molekülionen); HPLC-Säule: X Bridge *(Waters),* 2.1 x 50 mm, BEH 1.7 µm; Flussmittel: A: H2O/0.05% HC(O)OH, B: CH3CN/0.05% HC(O)OH. Gradient: 10-90% B in 1.7 min, 90% B für 0.2 min, 98-2% B in 0.6 min; Flussrate: 1.3 ml / min. Die Angabe *LC-MS (ZQ)* bezieht sich auf die Verwendung eines *Waters* ZQ4000 Gerätes und die Angabe *LC-MS (SQD)* auf die Benutzung eines Single Quadrupol API (Atomic Pressure lonization) Massendetektors *(Waters)* zur Aufnahme eines Massenspektrums.

### Verfahrensvariante 1

### Beispiel 1.1

### 2-(3-Methansulfonyl-phenyl)-thiazol-4-carbonsäure (3-carbamoyl-1-methyl-1H-pyrazol-4-yl)-amid

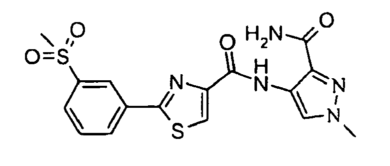

### Stufe a: Herstellung 2-(3-Methansulfonyl-phenyl)-thiazol-4-carbonsäureethylester

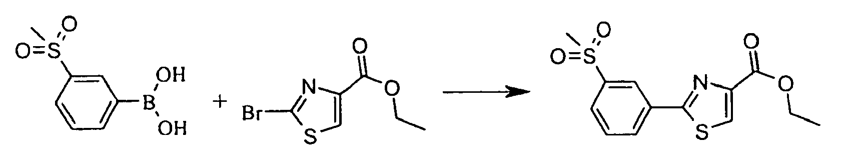

2-Bromthiazol-4-carbonsäureethylester (1 g), 3-(Methylsulfonyl)phenylboronsäure (1.02 g, 1.2 Äquivalente) und Kaliumcarbonat (1.76 g) werden in Dioxan (12.5 ml) vorgelegt. Man gibt (1,3-Diisopropylimidazol-2-yliden)(3-chlorpyridyl)palladium(II) dichlorid (288 mg) zu und die Mischung wird 18 h unter Schutzgas unter Rückfluß gerührt. Danach wird Dioxan am Rotationsverdampfer entfernt und der Rückstand mit Essigester und Wasser aufgenommen. Die organische Phase wird abgetrennt und die wässrige Phase zweimal mit Essigester nachextrahiert. Die vereinigten organischen Phasen werden mit Kochsalzlösung gewaschen und über Natriumsulfat getrocknet und eingeengt. Nach säulenchromatographischer Reinigung an Kieselgel (Essigester/Hexan) erhält man einen weißen Feststoff (629 mg).
C₁₂H₁₃NO₄S₂, M = 311.4. LC-MS (ZQ): Rₜ = 1.07, m/z = 312 [M+H]⁺.

### Stufe b: Herstellung 2-(3-Methansulfonyl-phenyl)-thiazol-4-carbonsäure

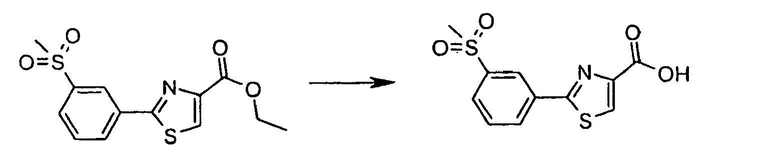

Das Produkt aus Stufe a (629 mg) in THF (12 ml) und Ethanol (12 ml) wird mit Natronlauge (1 M, 20 ml) versetzt und die Mischung wird 3 h bei Raumtemperatur gerührt. Durch Addition von wässriger Salzsäurelösung (1 M) wird die Lösung auf pH = 4 eingestellt. Danach entfernt man die organischen Lösemittel weitestgehend am Rotationsverdampfer und extrahiert die zurückbleibende Lösung mit Essigester, wäscht mit Kochsalzlösung und trocknet mit Natriumsulfat. Durch Einengen im Vakuum erhält man 2-(3-Methansulfonyl-phenyl)-thiazol-4-carbonsäure (241 mg) als Feststoff.
C₁₁H₉NO₄S₂, M = 283.0. LC-MS (ZQ): Rₜ = 1.00, m/z = 284 [M+H]⁺.
1H-NMR (300 MHz, D6-DMSO): 7.79 (m, 1H), 8.04 (m, 1H), 8.27 (m, 1H), 8.43 (m, 1H), 8.56 (s, 1H).

### Stufe c:

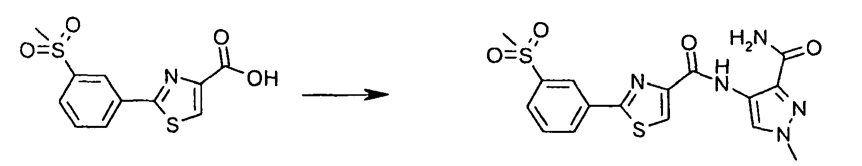

Eine Mischung aus 2-(3-Methansulfonyl-phenyl)-thiazol-4-carbonsäure (120 mg) aus Stufe b und 4-Amino-1-methyl-1H-pyrazol-3-carbonsäureamid (65 mg) in Triethylamin (176 µL) und DMF (3 ml) in Gegenwart von HATU (209 mg) wird bei Raumtemperatur 88 h gerührt. Man addiert Wasser, extrahiert mit Essigester und engt die organische Phase ein. Nach Reinigen durch präparative HPLC sowie säulenchromatographischer Reinigung an Kieselgel (Laufmittel Cyclohexan/Aceton) erhält man 2-(3-Methansulfonyl-phenyl)-thiazol-4-carbonsäure (3-carbamyl-1-methyl-1H-pyrazol-4-yl)-amid (3.4 mg) als weißen Feststoff.
C₁₆H₁₅N₅O₄S₂, M = 405.1; LC-MS (ZQ): Rₜ = 0.92, m/z = 406 [M+H]⁺.
1H-NMR (300 MHz, D6-DMSO): 3.31 (s, 3H), 3.90 (s, 3H), 7.56 (s, 1H), 7.72 (s, 1H), 7.84 (m, 1H), 8.07 (m, 1H), 8.32 - 8.38 (m, 2H), 8.43 (m, 1H), 8.55 (s, 1H), 11.3 (s, 1H)

### Verfahrensvariante 2

### Beispiel 2.1

### N-[5-(Aminocarbonyl)-1-methyl-1H-pyrazol-4-yl]-2-(2,3-dihydro-5-benzofuranyl)-4-thiazolcarboxamid

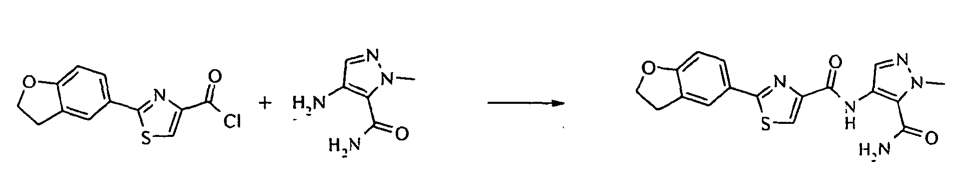

Man legt 4-Amino-2-methyl-2H-pyrazol-3-carbonsäureamid (35 mg) in Dichlormethan (1 ml) und Dimethylacetamid (0.5 ml) vor und addiert Triethylamin (50 Microliter) und 4-Dimethylaminopyridin (DMAP) (Spatelspitze) sowie 2-(2,3-Dihydro-1-benzofuran-5-yl)-1,3-thiazol-4-carbonylchlorid CAS 306936-10-7 (66 mg) und lässt bei Raumtemperatur 18.5 h rühren. Danach entfernt man die Lösemittel im Vakuum und erhält nach präparativer HPLC Beispiel 2.1 als weißen Feststoff (35 mg).
C₁₇H₁₅N₅O₃S, M = 369.4, LC-MS (ZQ): Rₜ = 1.01, m/z = 370 [M+H]⁺.

### Beispiel 2.2

### N-[2-(Aminocarbonyl)-4-methylphenyl]-2-(2,3-dihydro-5-benzofuranyl)-4-thiazolcarboxamid

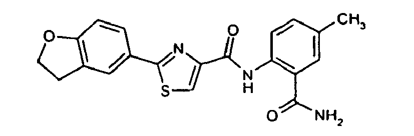

Analog zur Synthese von Beispiel 2.1 erhält man N-[2-(Aminocarbonyl)-4-methylphenyl]-2-(2,3-dihydro-5-benzofuranyl)-4-thiazolcarboxamid als Feststoff durch die Reaktion von 2-(2,3-Dihydro-1-benzofuran-5-yl)-1,3-thiazol-4-carbonylchlorid und 2-Amino-5-methylbenzamid.
C₂₀H₁₇N₃O₃S, M = 379.4, LC-MS (ZQ): Rₜ = 1.22, m/z = 380 [M+H]⁺.

### Beispiel 2.3

### N-[2-(Aminocarbonyl)-4-chlorphenyl]-2-(2,3-dihydro-5-benzofuranyl)-4-thiazolcarboxamid

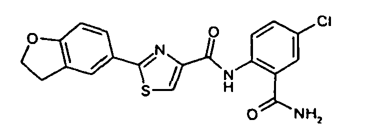

Analog zur Synthese von Beispiel 2.1 erhält man N-[2-(Aminocarbonyl)-4-chlorphenyl]-2-(2,3-dihydro-5-benzofuranyl)-4-thiazolcarboxamid als Feststoff durch die Reaktion von
2-(2,3-Dihydro-1-benzofuran-5-yl)-1,3-thiazol-4-carbonylchlorid und 2-Amino-5-chlorbenzamid.
C₁₉H₁₄CIN₃O₃S, M = 399.9. LC-MS (ZQ): Rₜ = 1.29 min, m/z = 400 [M+H]⁺.

### Beispiel 2.4

### N-[2-(Aminocarbonyl)phenyl]-2-(2,3-dihydro-5-benzofuranyl)-4-thiazolcarboxamid

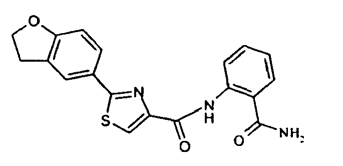

Analog zur Synthese von Beispiel 2.1 erhält man N-[2-(Aminocarbonyl)phenyl]-2-(2,3-dihydro-5-benzofuranyl)-4-thiazolcarboxamid als Feststoff durch die Reaktion von 2-(2,3-Dihydro-1-benzofuran-5-yl)-1,3-thiazol-4-carbonylchlorid und 2-Aminobenzamid.
C₁₉H₁₅N₃O₃S, M = 365.4. 1H-NMR (300 MHz, D6-DMSO): δ = 3.29 (t, 2H), 4.65 (t, 2H), 6.93 (m, 1H), 7.18 (m, 1H), 7.56 (m, 1H), 7.80 - 7.88 (m, 2H), 7.91 (m, 1H), 7.95 (s, 1H), 8.28 (s, 1H), 8.37 (s, 1H), 8.83 (m, 1H), 13.1 (s, 1H).

### Beispiel 2.5

### 2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid

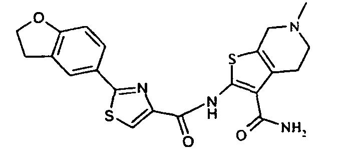

Analog zur Synthese von Beispiel 2.1 erhält man 2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid als Feststoff durch die Reaktion von 2-(2,3-Dihydro-1-benzofuran-5-yl)-1,3-thiazol-4-carbonylchlorid und 2-Amino-6-methyl-4,5,6,7-tetrahydrothieno[2,3-c]pyridin-3-carbonsäureamid.
C₂₁H₂₀N₄O₃S₂, M = 440.5. LC-MS: Rₜ = 2.98, m/z = 441.5 [M+H]⁺. Aufnahme durch ein Autopurifier HPLC-System der Firma *Waters* (1525µ Binary HPLC Pump, *(Waters),* Detector: MS: Micromass ZQ, Hersteller: *(Waters),* UV- Lampe: MUX UV 2488 Detector, Hersteller: *(Waters),* Wellenlänge: 210-350 nm, Säule: XBridge *(Waters),* 4,6x50 mm, C18 3,5µm, Gradient: Acetonitril / Wasser (enthält 0.1 % TFA), von: 1 % Acetonitril bis 99%, Runtime: 8 min., Flussrate:2 ml/min.

### Verfahrensvariante 3

### Beispiel 3.1

### N-[2-(Aminocarbonyl)phenyl]-2-(3,4-methylendioxyphenyl)-4-thiazolcarboxamid

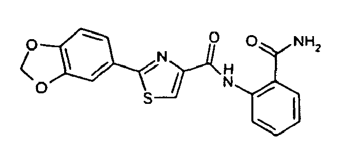

### a) Herstellung des Intermediates V.1

### N-[2-(Aminocarbonyl)phenyl]-2-brom-4-thiazolcarboxamid (Intermediat V.1)

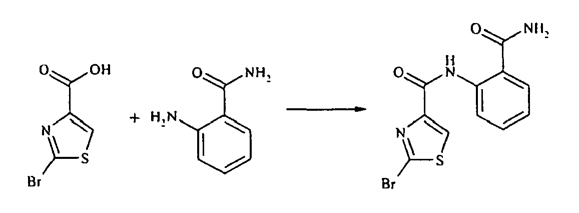

2-Brom-4-thiazolcarbonsäure (1 g), HATU (2.01 g) und 2-Aminobenzamid (0.65 g) in N,N-Dimethylformamid (DMF) (20 ml) werden vorgelegt. Man kühlt mit einem Eisbad ab und gibt N,N-Diisopropylethylamin (0.90 ml) zu. Man lässt 6 Tage bei Raumtemperatur rühren, gießt das Reaktionsgemisch auf Eiswasser, lässt unter Rühren auftauen und saugt den ausgefallenen Feststoff ab, wäscht zweimal mit Wasser, zweimal mit Diethylether und trocknet im Vakuum. Man erhält das Intermediat V.1 als Feststoff (1.4 g).
C₁₁H₈BrN₃O₂S, M = 326.2. 1H-NMR (300 MHz, D6-DMSO): δ = 7.20 (m, 1H), 7.56 (m, 1H), 7.79 (s, 1H), 7.84 (m, 1H), 8.30 (s, 1H), 8.47 (m, 1H), 8.67 (m, 1H), 12.9 (s, 1H).

### b) Herstellung des Endproduktes

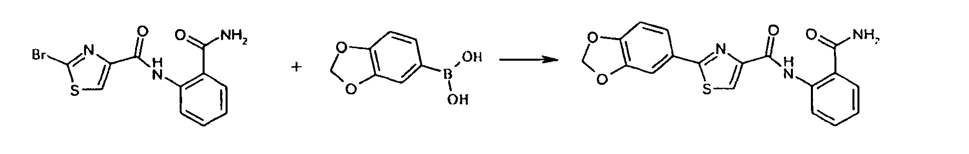

Das Intermediat V.1 (65 mg) und 3,4-Methylendioxyphenylboronsäure (46 mg, 1.4 Äquivalente) werden in Toluol (1.5 ml), Ethanol (1.5 ml) und wässriger Natriumcarbonatlösung (180 Microliter, 2M) vorgelegt. Man gibt Pd(PPh₃)₄ (23 mg) zu und erhitzt in der Microwelle (Gerät CEM Explorer, 120°C, 20 min, 300 Watt). Danach gibt man Wasser und Essigester zu, rührt um und trennt die organische Phase ab und engt diese in einer Zentrifuge ein. Der resultierende Feststoff wird durch präparative HPLC gereinigt.
C₁₈H₁₃N₃O₄S, M = 367.4, LC-MS (ZQ): Rₜ = 1.15, m/z = 368 [M+H]⁺.

### Beispiel 3.2

### N-[2-(Aminocarbonyl)phenyl]-2-(3-methylsulfonylphenyl)-4-thiazolcarboxamid

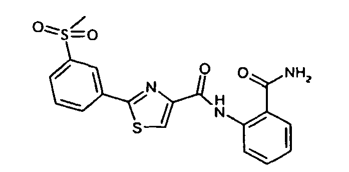

Man legt (analog zu C. J. O'Brien et. al. Chem. Eur. J. 2006, 12, 4743 - 4748) Intermediat V.1 (65 mg) und 3-Methylsulfonylphenylboronsäure (80 mg) in Dioxan (2 ml) vor und addiert Kaliumcarbonat (83 mg) und 1,3-Bis(2,6-diisopropylphenyl)imidazol-2-yliden(3-chlorpyridyl)palladium(II)dichlorid (10 mg). Nach Erhitzen in der Mikrowelle (120°C, 30 min, 300W) und Aufarbeitung wie bei der Beispiel 3.1 wird N-[2-(Aminocarbonyl)phenyl]-2-(3-methylsulfonylphenyl)-4-thiazolcarboxamid als weißer Feststoff erhalten.
C₁₈H₁₅N₃O₄S₂, M = 401.5. LC-MS (ZQ): R₁ = 1.01, m/z = 402 [M+H]⁺.

### Beispiel 3.3

### N-[2-(Aminocarbonyl)phenyl]-2-(3-cyanophenyl)-4-thiazolcarboxamid

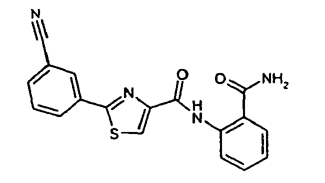

Analog zu Beispiel 3.2 erhält man N-[2-(Aminocarbonyl)phenyl]-2-(3-cyanophenyl)-4-thiazolcarboxamid aus Intermediat V.1 und 3-Cyanophenylboronsäure.
C₁₈H₁₂N₄O₂S, M = 348.4. LC-MS (ZQ): Rₜ = 1.11, m/z = 349 [M+H]⁺.

### Beispiel 3.4

### N-[2-(Aminocarbonyl)phenyl]-2-(3-fluoro-4-methoxyphenyl)-4-thiazolcarboxamid

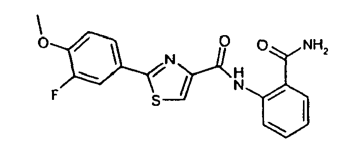

Analog Beispiel 3.2 erhält man N-[2-(Aminocarbonyl)phenyl]-2-(3-fluoro-4-methoxyphenyl)-4-thiazolcarboxamid aus Intermediat V.1 und 3-Fluoro-4-methoxyphenylboronsäure.
C₁₈H₁₄FN₃O₃S, M = 371.4. 1H-NMR (400 MHz, D6-DMSO): 3.91 (s, 3H), 7.15 (m, 1H), 7.29 (m, 1H), 7.53 (m, 1H), 7.77 -7.88 (3H), 7.94 (m, 1H), 8.26 (s, 1H), 8.41 (s, 1H), 8.70 (m, 1H), 13.2 (s, 1H).

### Beispiel 3.5

### N-[2-(Aminocarbonyl)-5-methylphenyl]-2-(4-methyl-3,4-dihydro-2H-benzo[1,4]oxazin-7-yl)-thiazol-4-carbonsäureamid

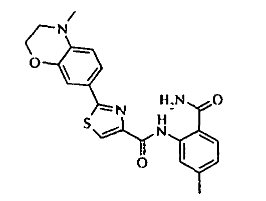

### a) Herstellung des Intermediates V.2

### N-[2-(Aminocarbonyl)-5-methylphenyl]-2-brom-4-thiazolcarboxamid (Intermediat V.2)

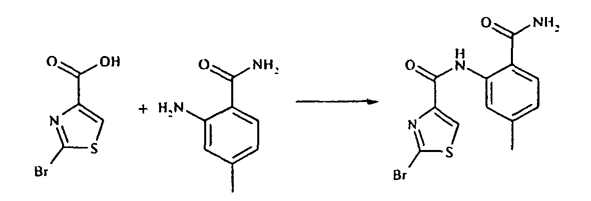

Analog zur Synthese von Intermediat V.1 wird 2-Brom-4-thiazolcarbonsäure und 2-Amino-4-methylbenzamid zu N-[2-(Aminocarbonyl)-5-methylphenyl]-2-brom-4-thiazolcarboxamid (Intermediat V.2) umgesetzt.
C₁₂H₁₀BrN₃O₂S, M = 339.0, LC-MS (ZQ): Rₜ = 1.04, m/z = 340 [M+H]⁺. 1H-NMR (300 MHz, D6-DMSO): δ = 2.32 (s, 3H), 6.97 (m, 1H) 7.65 (br. s., 1H), 7.70 (d, 1H), 8.18 (br. s., 1H), 8.40 (s, 1H), 8.49 (m, 1H), 13.0 (s, 1H).

### b) Herstellung des Endproduktes

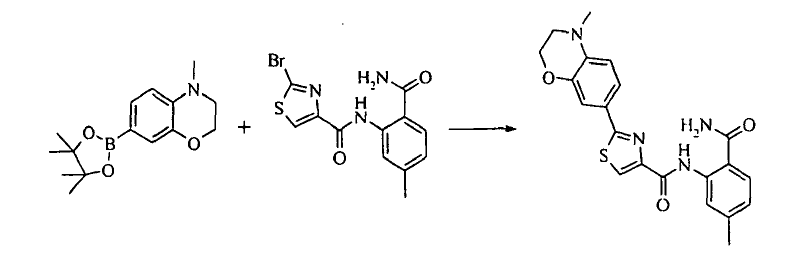

Man legt Intermediat V.2 (150 mg, 0.44 mmol) und 4-Methyl-7-(4,4,5,5-tetramethyl-1,3,2-dioxaborolan-2-yl)-3,4-dihydro-2H-1,4-benzoxazin (147 mg, 0.53 mmol) in Ethylenglykoldimethylether (3 ml) und wässriger Natriumhydrogencarbonatlösung (2 ml, 2M) vor, gibt Pd(PPh₃)₄ (51 mg, 0.044 mmol) zu und erhitzt in der Mikrowelle 45 min bei 110°C (300W) (Gerät CEM Explorer). Nach weiterer Zugabe von Pd(PPh₃)₄ (51 mg, 0.044 mmol) erhitzt man erneut 30 min bei 110°C. Das Lösemittel wird abdestilliert und der Rückstand in Essigester und Wasser aufgenommen, die organische Phase abgetrennt, die wässrige Phase mit Essigester extrahiert, die vereinigten organischen Phasen mit gesättigter Kochsalzlösung gewaschen und über Natriumsulfat getrocknet. Nach Einengen am Rotationsverdampfer wird der Rückstand per HPLC gereinigt.
C₂₁H₂₀N₄O₃S, M = 408.1, LC-MS (ZQ): Rₜ = 1.39, m/z = 409 [M+H]⁺. 1H-NMR (400 MHz, D6-DMSO): 2.33 (s, 3H), 2.91 (s, 3H), 3.33 (m, überlagert durch Wassersignal), 4.23 (m, 2H), 6.72 (m, 1H), 6.95 (m, 1H), 7.39 (m, 1H), 7.46 (m, 1H), 7.67 - 7.75 (m, 2H), 8.15 (br. s, 1H), 8.22 (s, 1H), 8.57 (s, 1H), 13. 1 (s, 1H).

### II. Biologische Wirkungen der erfindungsgemäßen Verbindungen

### TLR induzierte Zytokinfreisetzung in humanen "peripheral blood mononuclear cells" (PBMC)

### Versuchsprinzip

Aus humanem Vollblut isolierte PBMCs werden mit einem TLR Liganden stimuliert. Die Zytokin-Bestimmung wird mittels ELISA-Kits, eine Proliferations/Zellstoffwechselbestimmung wird mit Alamarblue durchgeführt.

### PBMC-Isolierung:

Für die Zellpräparation werden ca. 200 ml Blut mit einem Antikoagulanz (z.B. Citrat-Monovetten) versetzt. Pro Leucosep-Röhrchen werden 15ml Histopaque (Raumtemperatur, RT) eingefüllt und durch kurzes Anzentrifugieren (eine Minute bei 1000 x g, RT) durch die eingesetzte Fritte nach unten gedrückt. In die so vorbereiteten Röhrchen werden 20ml Blut gegeben und für 15 Minuten bei 800xg (RT) zentrifugiert. Nach der Zentrifugation ergibt sich von oben nach unten folgende Schichtung:
Plasma - PBMC - Histopaque - Filterscheibe - Histopaque - Erythrozyten und Granulozyten. Das überstehende Plasma wird abgesaugt. Die PBMC werden mitsamt dem darunterliegenden Histopaque in ein neues 50ml Röhrchen überführt, wobei immer der Inhalt von zwei Leucosep-Röhrchen
in ein 50ml Röhrchen gegeben wird. Die 50ml Röhrchen werden dann mit PBS auf 50ml aufgefüllt. Diese Zellsuspension wird für zehn Minuten bei 300xg (RT) zentrifugiert.
Der flüssige Überstand wird abgekippt und das Zellpellet mit wenig PBS resuspendiert und anschließend mit PBS auf 50ml aufgefüllt. Dieser Waschschritt wird zweimal wiederholt. Das entstandene Pellet wird in einem definierten Volumen von Medium (mit Zusätzen) aufgenommen. Zur Testung der Substanzen werden PBMC mit titrierten Konzentrationen der Testsubstanzen z.B. in Gegenwart oder Abwesenheit von TLR7 oder TLR9 Liganden für 18 Stunden inkubiert. Am nächsten Tag werden die Überstände mittels spezifischer ELISA auf den Gehalt von TNF-alpha oder anderen Zyto- oder Chemokinen untersucht. Die Stoffwechselaktivität der behandelten Zellen wird mithilfe von Alamarblue bestimmt.

### Ergebnisse:

| **Beispiel** | **EC₅₀** (TNF-α TLR7) |
|---|---|
| 1.1 | 8.5e-8 mol/L |
| 2.1 | 1.2e-6 mol/L |
| 2.5 | 1.1 e-6 mol/L |
| 3.1 | 3e-7 mol/L |
| 3.2 | 3.4e-6 mol/L |
| 3.4 | 1.2e-7 mol/L |

## Patentansprüche

1. Verbindungen der allgemeinen Formel (I) mit den Bausteinen A, B, C und D , 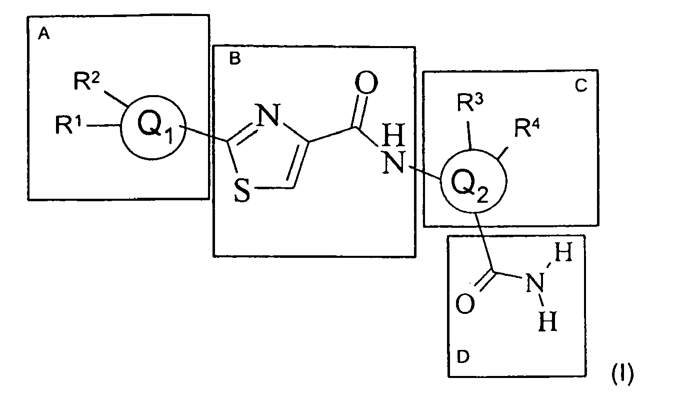 in der
die Bausteine B und D in ortho-Stellung zueinander stehen und
Q₁ für einen Phenylring oder hydrierten bicyclischen Heteroarylring steht, und
R¹ und R² unabhängig voneinander stehen für
(i) Wasserstoff, Hydroxy, Nitro, Halogen, Cyano, -CF₃, -NR⁵R⁶ oder
(ii) -O-SO₂-NR⁵R⁶, -SO₂-R⁷, -SO₂-NR⁵R⁶
(iii) -C(O)-OR¹⁰, -C(O)-R⁷, -C(O)-C₁-C₃-Fluoralkyl, -C(O)-NR⁵R⁶, -NH-C(O)-R⁷ oder
(iv) einen C₂-C₆ -Alkenyl-, C₂-C₆-Alkinyl-, C₁-C₆-Alkoxy-, C₁-C₆-Fluoralkyl- oder einen C₁-C₆-Fluoralkoxyrest, oder
(v) einen C₁-C₆-Alkylrest, wenn Q₁ für einen hydrierten bicyclischen Heteroarylring steht,
wobei (iv) und/oder (v) gegebenenfalls mit -C₁-C₃-Alkoxy, Hydroxy, -C(O)-OR¹⁰ oder -NR⁸R⁹ ein- oder mehrfach, gleich oder verschieden substituiert sein können, und
Q₂ für einen Aryl-, Heteroaryl- oder einen hydrierten bizyklischen Heteroarylring steht, und
R³ und R⁴ unabhängig voneinander stehen für
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷, wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷, oder
R⁵ und R⁶ bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
R⁷ für einen C₁-C₆ Alkylrest steht, und
R⁸, R⁹, R¹⁰ unabhängig voneinander stehen für Wasserstoff oder einen C₁-C₆ Alkylrest, und
R¹¹ und R¹² unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
sowie deren Salze, Enantiomere und Diastereomere.

2. Verbindungen der allgemeinen Formel (I) mit den Bausteinen A, B, C und D, in der
Q₁ für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl, Indanonyl- oder einen 2,3-Dihydroindolylring steht, und
R¹ und R² unabhängig stehen für
(i) Wasserstoff, Hydroxy, Halogen, Cyano, -CF₃, -NR⁵R⁶ oder
(ii) -SO₂-R⁷,
(iii) einen C₁-C₆-Alkoxy-, C₁-C₆-Fluoralkyl-, C₁-C₆-Fluoralkoxyrest oder
(iv) einen C₁-C₆-Alkylrest, wenn Q₁ für einen hydrierten bicyclischen Heteroarylring steht, und
Q₂ für einen Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht,
R³ und R⁴ unabhängig voneinander stehen für
(i) Wasserstoff, Halogen, -NR¹¹R¹² oder
(ii) einen C₁-C₆-Alkyl- oder C₁-C₆-Alkoxyrest,
jeweils gegebenenfalls mit Morpholin oder -NR⁸R⁹ substituiert, wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann, und
R⁷ für einen C₁-C₄ Alkylrest steht, und
R⁸ und R⁹ unabhängig voneinander stehen für Wasserstoff oder einen C₁-C₄ Alkylrest, und
R¹¹ und R¹² unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann,
sowie deren Salze, Enantiomere und Diastereomere.

3. Verbindungen der allgemeinen Formel (I) mit den Bausteinen A, B, C und D gemäß Anspruch 1 oder 2, in der
Q₁ für einen für Phenyl-, 3,4-Methylendioxyphenyl-, 2,3-Dihydrobenzofuranyl- oder einen 3,4-Dihydro-2H-benzo[1,4]oxazin-7-ylring steht,
sowie deren Salze, Enantiomere und Diastereomere.

4. Verbindungen der allgemeinen Formel (I) mit den Bausteinen A, B, C und D gemäß einem der Ansprüche 1 bis 3, in der
R¹ und R² unabhängig voneinander stehen für
(i) Wasserstoff, Halogen, Cyano oder
(ii) -SO₂-R⁷, oder
(iii) einen C₁-C₆-Alkoxyrest oder
(iv) einen C₁-C₆-Alkylrest, wenn Q₁ für einen hydrierten bicyclischen Heteroarylring steht,
wobei
R⁷ für einen C₁-C₃-Alkylrest steht,
sowie deren Salze, Enantiomere und Diastereomere.

5. Verbindungen der allgemeinen Formel (I) mit den Bausteinen A, B, C und D gemäß einem der Ansprüche 1 bis 4, in der
Q₂ für einen Phenyl-, Pyrazolyl- oder einen 4,5,6,7-Tetrahydro-thieno[2,3-c]pyridinylring steht,
sowie deren Salze, Enantiomere und Diastereomere.

6. Verbindungen der allgemeinen Formel (I) mit den Bausteinen A, B, C und D gemäß einem der Ansprüche 1 bis 5, in der
R³ und R⁴ unabhängig voneinander stehen für Wasserstoff, Halogen oder C₁-C₆-Alkyl,
sowie deren Salze, Enantiomere und Diastereomere.

7. Verbindungen der allgemeinen Formel (I) mit den Bausteinen A, B, C und D in der die Bausteine B und D in ortho-Stellung zueinander stehen und
Q₁ für einen für Phenyl-, 3,4-Methylendioxyphenyl-, 2,3-Dihydrobenzofuranyl- oder einen 3,4-Dihydro-2H-benzo[1,4]oxazin-7-ylring steht, und
R¹ und R² unabhängig voneinander stehen für
(i) Wasserstoff, Halogen, Cyano oder
(ii) -SO₂-R⁷.
(iii) einen C₁-C₆-Alkoxyrest oder
(iv) einen C₁-C₆-Alkylrest, wenn Q₁ für einen hydrierten bicyclischen Heteroarylring steht, und
Q₂ für einen für einen Phenyl-, Pyrazolyl- oder einen 4,5,6,7-Tetrahydro-thieno[2,3-c]pyridinylring steht, und
R³ und R⁴ unabhängig voneinander stehen für Wasserstoff, Halogen oder C₁-C₆-Alkyl,
wobei
R⁷ für einen C₁-C₃-Alkylrest steht,
sowie deren Salze, Enantiomere und Diastereomere.

8. Verbindungen nach einem der Ansprüche 1 bis 7, nämlich
2-(3-Methansulfonyl-phenyl)-thiazol-4-carbonsäure (3-carbamoyl-1-methyl-1H-pyrazol-4-yl)-amid
N-[5-(Aminocarbonyl)-1-methyl-1H-pyrazol-4-yl]-2-(2,3-dihydro-5-benzofuranyl)-4-thiazolcarboxamid
N-[2-(Aminocarbonyl)-4-methylphenyl]-2-(2,3-dihydro-5-benzofuranyl)-4-thiazolcarboxamid
N-[2-(Aminocarbonyl)-4-chlorphenyl]-2-(2,3-dihydro-5-benzofuranyl)-4-thiazolcarboxamid
N-[2-(Aminocarbonyl)phenyl]-2-(2,3-dihydro-5-benzofuranyl)-4-thiazolcarboxamid
2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid
N-[2-(Aminocarbonyl)phenyl]-2-(3,4-methylendioxyphenyl)-4-thiazolcarboxamid
N-[2-(Aminocarbonyl)phenyl]-2-(3-methylsulfonylphenyl)-4-thiazolcarboxamid
N-[2-(Aminocarbonyl)phenyl]-2-(3-cyanophenyl)-4-thiazolcarboxamid
N-[2-(Aminocarbonyl)phenyl]-2-(3-fluoro-4-methoxyphenyl)-4-thiazolcarboxamid
N-[2-(Aminocarbonyl)-5-methylphenyl]-2-(4-methyl-3,4-dihydro-2H-benzo[1,4]oxazin-7-yl)-thiazol-4-carbonsäureamid

9. Verfahren zur Herstellung von Verbindungen gemäß einem der Ansprüche 1 bis 8 **gekennzeichnet durch** die Umsetzung von Intermediaten der Formel (III) mit Intermediaten der Formel (II) mit Hilfe eines Amidkupplungsreagenzes. 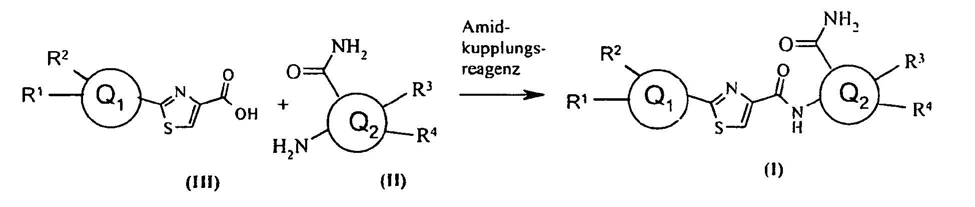 wobei
Q₁, Q₂, R¹, R², R³ und R⁴ die in der allgemeinen Formel (I) gemäß der Ansprüche 1 bis 7 angegebenen Bedeutungen besitzen.

10. Verfahren zur Herstellung von Verbindungen gemäß einem der Ansprüche 1 bis 8 **gekennzeichnet durch** die Umsetzung von Intermediaten der Formel (IV) mit Intermediaten der Formel (II) in Gegenwart einer Base. 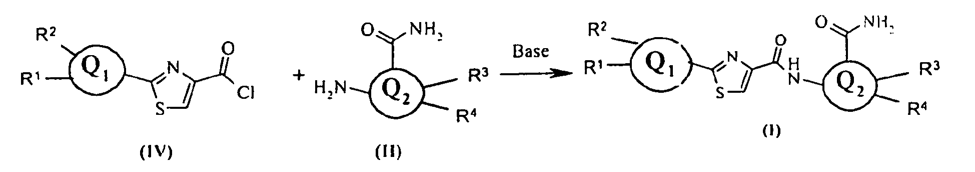 wobei
Q₁, Q₂, R¹, R², R³ und R⁴ die in der allgemeinen Formel (I) gemäß der Ansprüche 1 bis 7 angegebenen Bedeutungen besitzen.

11. Verfahren zur Herstellung von Verbindungen gemäß einem der Ansprüche 1 bis 8 mit den Teilschritten
a) Umsetzung von 2-Bromthiazol-4-carbonsäure mit Intermediaten der Formel (II) durch ein Amidkupplungsreagenz zu Intermediaten der Formel (V) 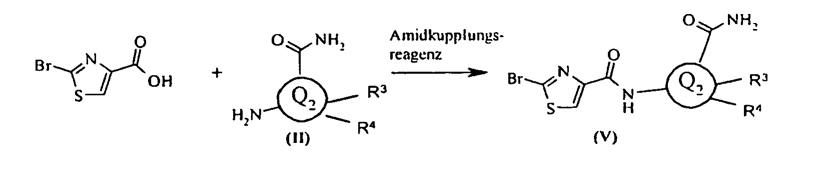
b) Umsetzung der Intermediate der Formel (V) mit Boronsäuren oder Boronsäurepinakolestern im Rahmen einer *Suzuki-Miyaura*-Reaktion zu Verbindungen der Formel (I) 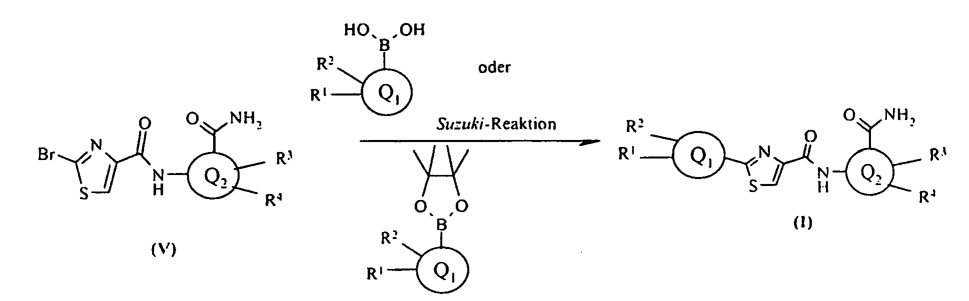 wobei
Q₁, Q₂, R¹, R², R³ und R⁴ die in der allgemeinen Formel (I) gemäß der Ansprüche 1 bis 7 angegebenen Bedeutungen besitzen.

12. Verbindungen gemäß einem der Ansprüche 1 bis 8 zur Verwendung als Arzneimittel.

13. Verwendung einer Verbindung gemäß einem der Ansprüche 1 bis 8 zur Herstellung eines Arzneimittels zur Behandlung von Erkrankungen, die mit entzündlichen, allergischen und / oder proliferativen Prozessen einhergehen.

14. Verbindungen gemäß einem der Ansprüche 1 bis 8 zur Verwendung als Arzneimittel gegen Erkrankungen, die mit entzündlichen, allergischen und / oder proliferativen Prozessen einhergehen.

15. Pharmazeutische Formulierung enthaltend eine Verbindung gemäß einem der Ansprüche 1 bis 8.

## Claims

1. Compounds of the general formula (I) with building blocks A, B, C and D 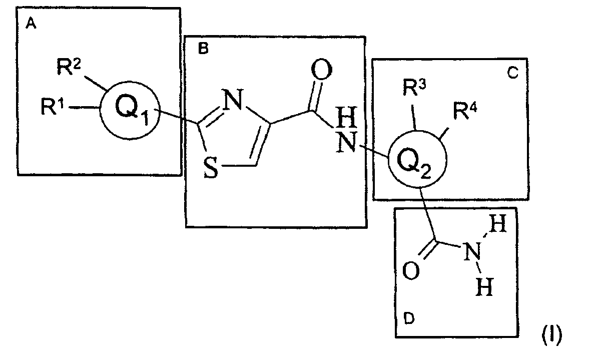 in which
the building blocks B and D are in ortho position relative to one another, and
Q₁ is a phenyl ring or hydrogenated bicyclic heteroaryl ring,
R¹ and R² are independently of one another
(i) hydrogen, hydroxy, nitro, halogen, cyano, -CF₃, -NR⁵R⁶ or
(ii) -O-SO₂-NR⁵R⁶, -SO2-R⁷, -SO₂-NR⁵R⁶,
(iii) -C(O)-OR¹⁰, -C(O)-R⁷, -C(O)-C₁-C₃-fluoroalkyl, -C(O)-NR⁵R⁶, -NH-C(O)-R⁷ or
(iv) a C₂-C₆-alkenyl, C₂-C₆-alkynyl, C₁-C₆-alkoxy, C₁-C₆-fluoroalkyl or a C₁-C₆-fluoroalkoxy radical, or
(v) a C₁-C₆-alkyl radical if Q₁ is a hydrogenated bicyclic heteroaryl ring,
where (iv) and/or (v) may optionally be substituted one or more times, identically or differently, by -C₁-C₃-alkoxy, hydroxy, -C(O)-OR¹⁰ or - NR⁸R⁹ and
Q₂ is an aryl, heteroaryl or a hydrogenated bicyclic heteroaryl ring; and
R³ and R⁴ are independently of one another
(i) hydrogen, halogen or -NR¹¹R¹², or
(ii) a C₁-C₆-alkyl or C₁-C₆-alkoxy radical which are in each case optionally substituted one or more times, identically or differently, by hydroxy, C₁-C₃-alkoxy, -NR⁸R⁹ or heterocyclyl, where the heterocyclyl ring may be substituted one or more times, identically or differently, by C₁-C₃-alkyl or -C(O)-R⁷, where
R⁵ and R⁶ are independently of one another hydrogen or a C₁-C₆-alkyl radical which is optionally substituted one or more times, identically or differently, by hydroxy, -C₁-C₃-alkoxy, - NR⁸R⁹ or heterocyclyl, where the heterocyclyl ring may be substituted one or more times, identically or differently, by C₁-C₃-alkyl or -C(O)-R⁷, or
R⁵ and R⁶ form alternatively together with the nitrogen atom a 5- to 7-membered ring which optionally comprises a further heteroatom in addition to the nitrogen atom, and which is optionally substituted one or more times, identically or differently, by C₁-C₆-alkyl and/or by -C(O)-R⁷, and
R⁷ is a C₁-C₆-alkyl radical, and
R⁸, R⁹, R¹⁰ are independently of one another hydrogen or a C₁-C₆-alkyl radical, and
R¹¹ and R¹² are independently of one another hydrogen or a C₁-C₆-alkyl radical which is optionally substituted one or more times, identically or differently, by hydroxy, -C₁-C₃-alkoxy, - NR⁸R⁹ or heterocyclyl, where the heterocyclyl ring may be substituted one or more times, identically or differently, by C₁-C₃-alkyl or -C(O)-R⁷,
and the salts, enantiomers and diastereomers thereof.

2. Compounds of the general formula (I) with building blocks A, B, C and D, in which
Q₁ is a phenyl, benzodioxolyl, 3,4-methylenedioxyphenyl, 2,3-dihydrobenzofuranyl, 3,4-dihydro-2H-benzo[1,4]oxazin-7-yl, indanonyl or a 2,3-dihydroindolyl ring, and
R¹ and R² are independently
(i) hydrogen, hydroxy, halogen, cyano, -CF₃, -NR⁵R⁶ or
(ii) -SO₂R⁷
(iii) a C₁-C₆-alkoxy, C₁-C₆-fluoroalkyl, C₁-C₆-fluoroalkoxy radical or
(iv) a C₁-C₆-alkyl radical if Q₁ is a hydrogenated bicyclic heteroaryl ring, and
Q₂ is a phenyl, thienyl, thiazolyl, furanyl, pyrrolyl, oxazolyl, imidazolyl, pyrazolyl, isoxazolyl, isothiazolyl, 1,2,3-triazolyl, pyridinyl, pyridazinyl, pyrimidinyl, pyrazinyl, 1,2,3,4-tetrahydroisoquinolinyl, 1,2,3,4-tetrahydroquinolinyl, 2,3-dihydro-1H-indolyl, 2,3-dihydro-1H-isoindolyl, 4,5,6,7-tetrahydrothieno-[2,3-c]pyridinyl, 4,5,6,7-tetrahydrothieno[3,2-c]pyridinyl, 2,3-dihydrobenzofuranyl, benzo[1,3]dioxolyl, 2,3-dihydrobenzo[1,4]dioxinyl, 1,2,3,4-tetrahydroquinoxalinyl or a 3,4-dihydro-2H-benzo[1,4]oxazinyl ring,
R³ and R⁴ are independently of one another
(i) hydrogen, halogen, -NR¹¹R¹² or
(ii) a C₁-C₆-alkyl or C₁-C₆-alkoxy radical, in each case optionally substituted by morpholine or -NR⁸R⁹,
where
R⁵ and R⁶ are independently of one another hydrogen or a C₁-C₆ alkyl radical which may optionally be substituted one or more times, identically or differently, by hydroxy, -NR⁸R⁹, halogen or C₁-C₃-alkoxy, and
R⁷ is a C₁-C₄ alkyl radical, and
R⁸ and R⁹ are independently of one another hydrogen or a C₁-C₄ alkyl radical, and
R¹¹ and R¹² are independently of one another hydrogen or a C₁-C₆ alkyl radical which may optionally be substituted one or more times, identically or differently, by morpholine or by-N(CH₃)₂, -NH-CH₃ or -NH-C₂H₅,
and the salts, enantiomers and diastereomers thereof.

3. Compounds of the general formula (I) with building blocks A, B, C and D according to Claim 1 or 2, in which
Q₁ is a phenyl, 3,4-methylenedioxyphenyl, 2,3-dihydrobenzofuranyl or a 3,4-dihydro-2H-benzo[1,4]oxazin-7-yl ring,
and the salts, enantiomers and diastereomers thereof.

4. Compounds of the general formula (I) with building blocks A, B, C and D according to any of Claims 1 to 3, in which
R¹ and R² are independently of one another
(i) hydrogen, halogen, cyano or
(ii) -SO₂R, or
(iii) a C₁-C₆-alkoxy radical or
(iv) a C₁-C₆-alkyl radical if Q₁ is a hydrogenated bicyclic heteroaryl ring,
where R⁷ is a C₁-C₃-alkyl radical,
and the salts, enantiomers and diastereomers thereof.

5. Compounds of the general formula (I) with building blocks A, B, C and D according to any of Claims 1 to 4, in which
Q₂ is a phenyl, pyrazolyl, or a 4,5,6,7-tetrahydrothieno[2,3-c]pyridinyl ring, and the salts, enantiomers and diastereomers thereof.

6. Compounds of the general formula (I) with building blocks A, B, C and D according to any of Claims 1 to 5, in which
R³ and R⁴ are independently of one another hydrogen, halogen or C₁-C₆-alkyl,
and the salts, enantiomers and diastereomers thereof.

7. Compounds of the general formula (I) with building blocks A, B, C and D in which
the building blocks B and D are in ortho position relative to one another, and
Q₁ is a phenyl, 3,4-methylenedioxyphenyl, 2,3-dihydrobenzofuranyl of a 3,4-dihydro-2H-benzo[1,4]oxazin-7yl ring, and
R¹ and R² are independently of one another
(i) hydrogen, halogen, cyano or
(ii) -SO₂-R⁷
(iii) a C₁-C₆-alkoxy radical or
(iv) a C₁-C₆-alkyl radical if Q₁ is a hydrogenated bicyclic heteroaryl ring, and
Q₂ is a phenyl, pyrazolyl or a 4,5,6,7-tetrahydrothieno[2,3-c]pyridinyl ring, and
R³ and R⁴ are independently of one another hydrogen, halogen or C₁-C₆-alkyl,
where
R⁷ is a C₁-C₃-alkyl radical,
and the salts, enantiomers and diastereomers thereof.

8. Compounds according to any of Claims 1 to 7, namely
2-(3-Methanesulphonylphenyl)-thiazole-4-(3-carbamoyl-1-methyl-1H-pyrazol-4-yl)-carboxamide
N-[5-(Aminocarbonyl)-1-methyl-1H-pyrazol-4-yl]-2-(2,3-dihydro-5-benzofuranyl)-4-thiazolecarboxamide
N-[2-(Aminocarbonyl)-4-methylphenyl]-2-(2,3-dihydro-5-benzofuranyl)-4-thiazolecarboxamide
N-[2-(Aminocarbonyl)-4-chlorophenyl]-2-(2,3-dihydro-5-benzofuranyl)-4-thiazolecarboxamide
N-[2-(Aminocarbonyl)phenyl]-2-(2,3-dihydro-5-benzofuranyl)-4-thiazolecarboxamide
2-{[2-(2,3-Dihydrobenzofuran-5-yl)-thiazole-4-carbonyl]-amino}-6-methyl-4,5,6,7-tetrahydrothieno[2,3-c]pyridine-3-carboxamide
N-[2-(Aminocarbonyl)phenyl]-2-(3,4-methylenedioxyphenyl)-4-thiazolecarboxamide
N-[2-(Aminocarbonyl)phenyl]-2-(3-methylsulphonylphenyl)-4-thiazolecarboxamide
N-[2-(Aminocarbonyl)phenyl]-2-(3-cyanophenyl)-4-thiazolecarboxamide
N-[2-(Aminocarbonyl)phenyl]-2-(3-fluoro-4-methoxyphenyl)-4-thiazolecarboxamide
N-[2-(Aminocarbonyl)-5-methylphenyl]-2-(4-methyl-3,4-dihydro-2H-benzo[1,4]oxazin-7-yl)-thiazole-4-carboxamide

9. Process for preparing compounds according to any of Claims 1 to 8 **characterized by** the reaction of intermediates of the formula (III) with intermediates of the formula (II) with the aid of an amide coupling reagent 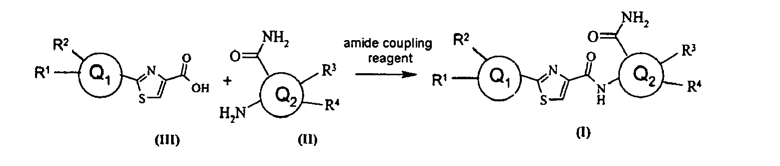 where
Q₁, Q₂, R¹, R², R³ and R⁴ have the meanings indicated in the general formula (I) according to Claims 1 to 7.

10. Process for preparing compounds according to any of Claims 1 to 8 **characterized by** the reaction of intermediates of the formula (IV) with intermediates of the formula (II) in the presence of a base 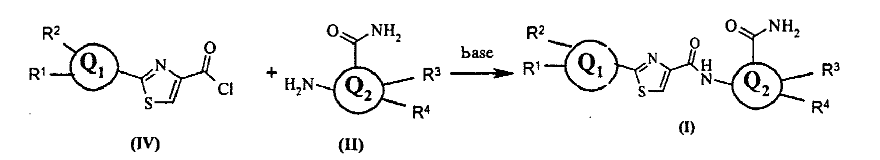 where
Q₁, Q₂, R¹, R², R³ and R⁴ have the meanings indicated in the general formula (I) according to Claims 1 to 7.

11. Process for preparing compounds according to any of Claims 1 to 8 having the substeps
a) reaction of 2-bromothiazole-4-carboxylic acid with intermediates of the formula (II) by an amide coupling reagent to give intermediates of the formula (v) 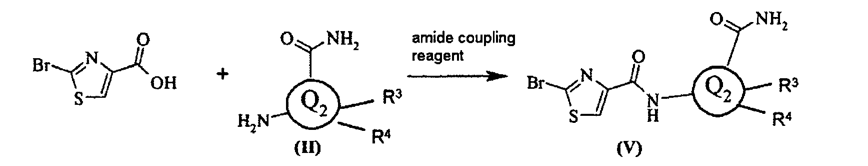
b) reaction of the intermediates of the formula (V) with boronic acids or boronic acid pinacol esters in a *Suzuki-Miyaura* reaction to give compounds of the formula (I) 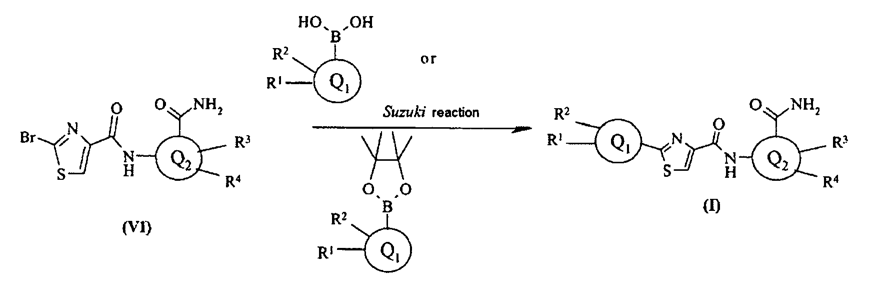 where
Q₁, Q₂, R¹, R², R³ and R⁴ have the meanings indicated in the general formula (I) according to Claims 1 to 7.

12. Compounds according to any of Claims 1 to 8 for use as medicaments.

13. Use of a compound according to any of Claims 1 to 8 for the production of a medicament for the treatment of diseases which involve inflammatory, allergic and/or proliferative processes.

14. Compounds according to any of Claims 1 to 8 for use as medicaments for treating disorders associated with inflammatory, allergic and/or proliferative processes.

15. Pharmaceutical formulation comprising a compound according to any of Claims 1 to 8.

## Revendications

1. Composés de formule générale (I) présentant les éléments constitutifs A, B, C et D, 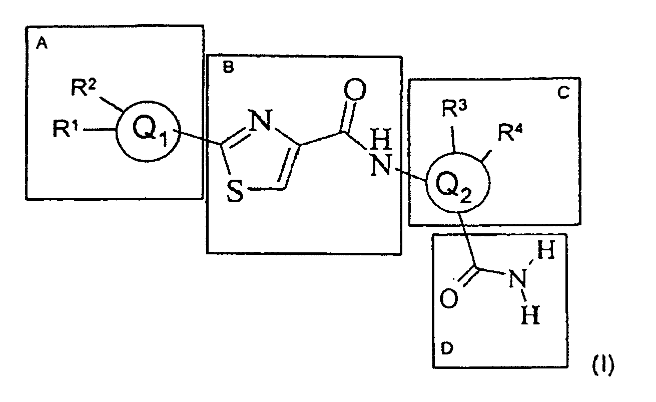 dans laquelle
les éléments constitutifs B et D se trouvent en position ortho l'un par rapport à l'autre et
Q₁ représente un cycle phényle ou un cycle hétéroaryle bicyclique hydrogéné, et
R¹ et R² représentent indépendamment l'un de l'autre
(i) un atome d'hydrogène ou d'halogène, ou un groupe hydroxy, nitro, cyano, -CF₃, -NR⁵R⁶ ou
(ii) -O-SO₂-NR⁵R⁶, -SO₂-R⁷, -SO₂NR⁵R⁶
(iii) -C(O)-OR¹⁰, -C(O)-R⁷, -C(O)-fluoroalkyle(C₁-C₃), -C(O)-NR⁵R⁶, -NH-C(O)-R⁷ ou
(iv) un radical alcényle en C₂-C₆, alcynyle en C₂-C₆, alcoxy en C₁-C₆, fluoroalkyle en C₁-C₆, ou un radical fluoroalcoxy(C₁-C₆), ou
(v) un radical alkyle en C₁-C₆, lorsque Q₁ représente un cycle hétéroaryle bicyclique hydrogéné,
les radicaux (iv) et/ou (v) pouvant éventuellement porter un ou plusieurs substituants, identiques ou différents, alcoxy en C₁-C₃, hydroxy, -C(O)-OR¹⁰ ou -NR⁸R⁹, et
Q₂ représente un cycle aryle, hétéroaryle ou un cycle hétéroaryle bicyclique hydrogéné, et
R³ et R⁴ représentent, indépendamment l'un de l'autre,
(i) un atome d'hydrogène, d'halogène ou -NR¹¹R¹², ou
(ii) un radical alkyle en C₁-C₆ ou alcoxy en C₁-C₆, qui portent éventuellement chacun un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷,
R⁵ et R⁶ représentant, indépendamment l'un de l'autre, un atome d'hydrogène ou un radical alkyle en C₁-C₆ qui porte éventuellement un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷, ou
R⁵ et R⁶ pouvant également former ensemble, avec l'atome d'azote, un cycle à 5-7 chaînons, qui éventuellement contient en plus de l'atome d'azote un autre hétéroatome et qui porte éventuellement un ou plusieurs substituants, identiques ou différents, alkyle en C₁-C₆ et/ou -C(O)-R⁷, et
R⁷ représentant un radical alkyle en C₁-C₆, et
R⁸, R⁹, R¹⁰ représentant chacun indépendamment un atome d'hydrogène ou un radical alkyle en C₁-C₆, et
R¹¹ et R¹² représentant, indépendamment l'un de l'autre, un atome d'hydrogène ou un radical alkyle en C₁-C₆ qui porte éventuellement un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷
ainsi que leurs sels, énantiomères et diastéréoisomères.

2. Composés de formule générale (I) présentant les éléments constitutifs A, B, C et D,
dans lesquels
Q₁ représente un cycle phényle, benzodioxolyle, 3,4-méthylènedioxyphényle, 2,3-dihydrobenzofurannyle, 3,4-dihydro-2H-benzo[1,4]oxazin-7-yle, indanonyle ou un cycle 2,3-dihydroindolyle, et
R¹ et R² représentent indépendamment l'un de l'autre
(i) un atome d'hydrogène ou d'halogène, ou un groupe hydroxy, cyano, -CF₃, -NR⁵R⁶ ou
(ii) -SO₂-R⁷,
(iii) un radical alcoxy en C₁-C₆, fluoroalkyle (C₁-C₆), fluoroalcoxy(C₁-C₆) ou
(iv) un radical alkyle en C₁-C₆ lorsque Q₁ représente un cycle hétéroaryle bicyclique hydrogéné, et
Q₂ représente un cycle phényle, thiényle, thiazolyle, furannyle, pyrrolyle, oxazolyle, imidazolyle, pyrazolyle, isoxazolyle, isothiazolyle, 1,2,3-triazolyle, pyridinyle, pyridazinyle, pyrimidinyle, pyrazinyle, 1,2,3,4-tétrahydro-isoquinolinyle, 1,2,3,4-tétrahydroquinolinyle, 2,3-dihydro-1H-indolyle, 2,3-dihydro-1H-iso-indolyle, 4,5,6,7-tétrahydro-thiéno[2,3-c]pyridinyle, 4,5,6,7-tétrahydro-thiéno[3,2-c]pyridinyle, 2,3-dihydro-benzofurannyle, benzo[1,3]dioxolyle, 2,3-dihydro-benzo[1,4]dioxinyle, 1,2,3,4-tétrahydro-quinoxalinyle ou un cycle 3,4-dihydro-2H-benzo[1,4]oxazinyle,
R³ et R⁴ représentent, indépendamment l'un de l'autre,
(i) un atome d'hydrogène, d'halogène, -NR¹¹R¹², ou
(ii) un radical alkyle ou alcoxy en C₁-C₆, éventuellement substitués chacun par morpholine ou -NR⁸R⁹,
R⁵ et R⁶ représentant, indépendamment l'un de l'autre, un atome d'hydrogène ou un radical alkyle en C₁-C₆ qui porte éventuellement un ou plusieurs substituants, identiques ou différents, hydroxy, -NR⁸R⁹, halogène ou alcoxy en C₁-C₃, et
R⁷ représentant un radical alkyle en C₁-C₄, et
R⁸ et R⁹ représentent, indépendamment l'un de l'autre, un atome d'hydrogène ou un radical alkyle en C₁-C₄, et
R¹¹ et R¹² représentant, indépendamment l'un de l'autre, un atome d'hydrogène ou un radical alkyle en C₁-C₆ qui porte éventuellement un ou plusieurs substituants, identiques ou différents, morpholine ou -N(CH₃)₂, -NH-CH₃ ou -NH-C₂H₅,
ainsi que leurs sels, énantiomères et diastéréoisomères.

3. Composés de formule générale (I) présentant les éléments constitutifs A, B, C et D selon la revendication 1 ou 2, dans lesquels
Q₁ représente un cycle phényle, 3,4-méthylènedioxyphényle, 2,3-dihydrobenzofurannyle ou un cycle 3,4-dihydro-2H-benzo[1,4]oxazin-7-yle,
ainsi que leurs sels, énantiomères et diastéréoisomères.

4. Composés de formule générale (I) présentant les éléments constitutifs A, B, C et D selon l'une quelconque des revendications 1 à 3, dans lesquels
R¹ et R² représentent indépendamment l'un de l'autre
(i) un atome d'hydrogène ou d'halogène, un groupe cyano ou
(ii) -SO₂-R⁷, ou
(iii) un radical alcoxy en C₁-C₆ ou
(iv) un radical alkyle en C₁-C₆ lorsque Q₁ représente un cycle hétéroaryle bicyclique hydrogéné,
R⁷ représentant un radical alkyle en C₁-C₃,
ainsi que leurs sels, énantiomères et diastéréoisomères.

5. Composés de formule générale (I) présentant les éléments constitutifs A, B, C et D selon l'une quelconque des revendications 1 à 4, dans lesquels
Q₂ représente un cycle phényle, pyrazolyle ou un cycle 4,5,6,7-tétrahydro-thiéno[2,3-c]pyridinyle,
ainsi que leurs sels, énantiomères et diastéréoisomères.

6. Composés de formule générale (I) présentant les éléments constitutifs A, B, C et D, selon l'une quelconque des revendications 1 à 5, dans lesquels
R³ et R⁴ représentent, indépendamment l'un de l'autre, un atome d'hydrogène, d'halogène ou un groupe alkyle en C₁-C₆,
ainsi que leurs sels, énantiomères et diastéréoisomères.

7. Composés de formule générale (I) présentant les éléments constitutifs A, B, C et D, dans lesquels les éléments constitutifs B et D se trouvent en position ortho l'un par rapport à l'autre et
Q₁ représente un cycle phényle, 3,4-méthylènedioxyphényle, 2,3-dihydrobenzofurannyle ou un cycle 3,4-dihydro-2H-benzo[1,4]oxazin-7-yle, et
R¹ et R² représentent indépendamment l'un de l'autre
(i) un atome d'hydrogène ou d'halogène, un groupe cyano ou
(ii) -SO₂-R⁷,
(iii) un radical alcoxy en C₁-C₆ ou
(iv) un radical alkyle en C₁-C₆ lorsque Q₁ représente un cycle hétéroaryle bicyclique hydrogéné, et
Q₂ représente un cycle phényle, pyrazolyle ou un cycle 4,5,6,7-tétrahydro-thiéno[2,3-c]-pyridinyle, et
R³ et R⁴ représentent, indépendamment l'un de l'autre, un atome d'hydrogène, d'halogène ou un groupe alkyle en C₁-C₆,
R⁷ représentant un radical alkyle en C₁-C₃,
ainsi que leurs sels, énantiomères et diastéréoisomères.

8. Composés selon l'une quelconque des revendications 1 à 7, à savoir
(3-carbamoyl-1-méthyl-1H-pyrazol-4-yl)-amide d'acide 2-(3-méthanesulfonyl-phényl)-thiazole-4-carboxylique
N-[5-(aminocarbonyl)-1-méthyl-1H-pyrazol-4-yl]-2-(2,3-dihydro-5-benzofurannyl)-4-thiazolecarboxamide
N-[2-(aminocarbonyl)-4-chlorophényl]-2-(2,3-dihydro-5-benzofurannyl)-4-thiazolecarboxamide
N-[2-(aminocarbonyl)-4-méthylphényl]-2-(2,3-dihydro-5-benzofurannyl)-4-thiazolecarboxamide
N-[2-(aminocarbonyl)phényl]-2-(2,3-dihydro-5-benzofurannyl)-4-thiazolecarboxamide
2-{[2-(2,3-dihydro-benzofurann-5-yl)-thiazole-4-carbonyl]-amino}-6-méthyl-4,5,6,7-tétrahydrothiéno[2,3-c]pyridine-3-carboxamide
N-[2-(aminocarbonyl)phényl]-2-(3,4-méthylènedioxyphényl)-4-thiazolecarboxamide
N-[2-(aminocarbonyl)phényl]-2-(3-méthylsulfonylphényl)-4-thiazolecarboxamide
N-[2-(aminocarbonyl)phényl]-2-(3-cyanophényl)-4-thiazolecarboxamide
N-[2-(aminocarbonyl)phényl]-2-(3-fluoro-4-méthoxyphényl)-4-thiazolecarboxamide
N-[2-(aminocarbonyl)-5-méthylphényl]-2-(4-méthyl-3,4-dihydro-2H-benzo[1,4]oxazin-7-yl)-thiazole-4-carboxamide.

9. Procédé pour la préparation de composés selon l'une quelconque des revendications 1 à 8, **caractérisé par** la mise en réaction de composés intermédiaires de formule (III) avec des composés intermédiaires de formule (II) à l'aide d'un réactif de couplage d'amides 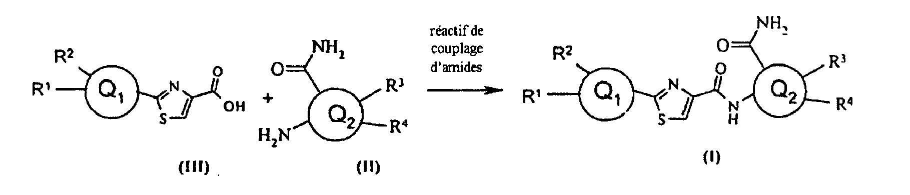 Q₁, Q₂, R¹, R², R³ et R⁴ ayant les significations indiquées dans la formule générale (I) selon les revendications 1 à 7.

10. Procédé pour la préparation de composés selon l'une quelconque des revendications 1 à 8, **caractérisé par** la mise en réaction de composés intermédiaires de formule (IV) avec des composés intermédiaires de formule (II) en présence d'une base 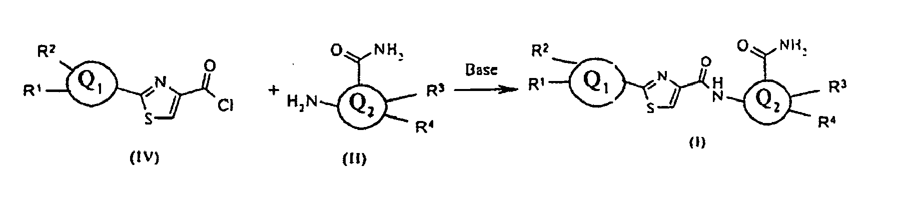 Q₁, Q₂, R¹, R², R³ et R⁴ ayant les significations indiquées dans la formule générale (I) selon les revendications 1 à 7.

11. Procédé pour la préparation de composés selon l'une quelconque des revendications 1 à 8, comportant les étapes
a) mise en réaction d'acide 2-bromothiazole-4-carboxylique avec des composés intermédiaires de formule (II) à l'aide d'un réactif de couplage d'amides de formule (V) 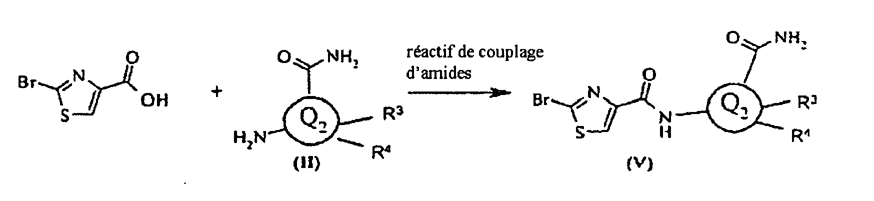
b) mise en réaction des composés intermédiaires de formule (V) avec des acides boroniques ou des boronates de pinacol dans le cadre d'une réaction de Suzuki-Miyaura, pour l'obtention de composés de formule (I) 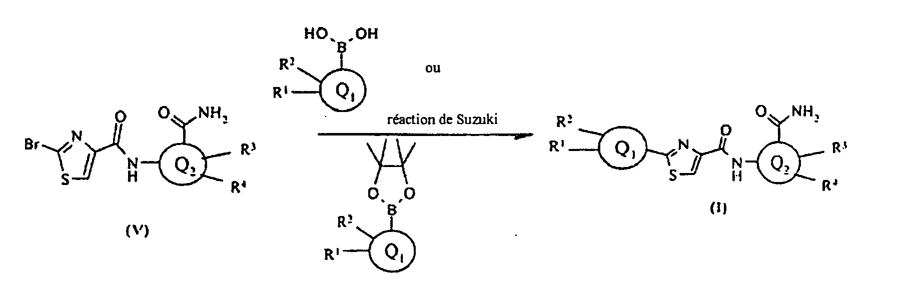 Q₁, Q₂, R¹, R², R³ et R⁴ ayant les significations indiquées dans la formule générale (I) selon les revendications 1 à 7.

12. Composés selon l'une quelconque des revendications 1 à 8, pour utilisation en tant que médicament.

13. Utilisation d'un composé selon l'une quelconque des revendications 1 à 8, pour la fabrication d'un médicament destiné au traitement de maladies qui s'accompagnent de processus inflammatoires, allergiques et/ou prolifératifs.

14. Composés selon l'une quelconque des revendications 1 à 8, pour utilisation en tant que médicament contre des maladies qui s'accompagnent de processus inflammatoires, allergiques et/ou prolifératifs.

15. Composition pharmaceutique contenant un composé selon l'une quelconque des revendications 1 à 8.
